# EUROPEAN PATENT SPECIFICATION

(11) **EP 1 521 827 B1**
(45) Date of publication and mention of the grant of the patent: **18.08.2010**
(21) Application number: 03763976.2
(22) Date of filing: 09.07.2003
(51) Int. Cl.: C12N 15/00, A01K 67/027, C12N 5/16

(54) **MODULATION OF CYTOCHROME P450 REDUCTASE ACTIVITY**
MODULIERUNG DER CYTOCHROME P540 REDUKTASE AKTIVITÄT
MODULATION DE L'ACTIVITE DE LA CYTOCHROME P450 REDUCTASE

(30) Priority: 12.07.2002 GB 0216203
(43) Date of publication of application: 13.04.2005
(60) Divisional application: 09155656.3
(73) Proprietor: Cancer Research Technology Limited, London WC2A 3NL (GB)
(72) Inventor: WOLF, Charles Roland, CXR Biosciences Limited, Dundee DD1 5JJ (GB); HENDERSON, Colin J., Cancer Res. UK Molecular, Dundee DD1 9SY (GB)
(74) Representative: Harrison Goddard Foote
(86) International application number: PCT/GB2003/002967
(87) International publication number: WO 2004/007708

(56) References cited:
- DING X ET AL: "A Mouse Model with Liver-Specific Deletion of the NADPH-Cytochrome P450 Reductase Gene" TOXICOLOGICAL SCIENCES, vol. 72, no. S-1, March 2003 (2003-03), page 337 XP009022989
- HENDERSON C J ET AL: "Inactivation of the Hepatic Cytochrome P450 System by Conditional Deletion of Hepatic Cytochrome P450 Reductase" THE JOURNAL OF BIOLOGICAL CHEMISTRY, vol. 278, no. 15, 11 April 2003 (2003-04-11), pages 13480-13486, XP001156584
- WU J ET AL: "Conditional Knockout of the Mouse NADPH-Cytochrome P450 Reductase Gene" GENESIS, vol. 36, 31 July 2003 (2003-07-31), pages 177-181, XP009022993
- GU J ET AL: "Liver-specific Deletion of the NADPH-Cytochrome P450 Reductase Gene" THE JOURNAL OF BIOLOGICAL CHEMISTRY, vol. 278, no. 28, 11 July 2003 (2003-07-11), pages 25895-25901, XP001156745
- SHEN A L ET AL: "Association of Multiple Developmental Defects and Embryonic Lethality with Loss of Microsomal NADPH-Cytochrome P450 Oxidoreductase" THE JOURNAL OF BIOLOGICAL CHGEMISTRY, vol. 277, no. 8, 22 February 2002 (2002-02-22), pages 6536-6541, XP001156823

## Description

The present invention relates to the uses of non-human transgenic animals, tissues and/or cells derived therefrom having depleted or ablated cytochrome P450 reductase (CPR) expression, methods of producing such animals, tissues and/or cells, for use especially, but non exclusively, in both *in vivo* and *in vitro* screening of therapeutic agents, drug development and drug metabolism/disposition studies.

### Background to the Invention

Transgenic animals are characterised by the presence of exogenous or non-native DNA integrated into their genetic information. The animals express or do not express, as the case may be, the genetic characteristic encoded by the foreign DNA.

The hepatic cytochrome P450-dependent monoxygenase system plays a central role in mammalian defence against harmful environmental chemicals; it is also a major determinant of the half-life and pharmacological properties of therapeutic drugs and in certain cases, mediates the activation of drugs, toxins and carcinogens to their ultimate toxic species. Several other functions have been ascribed to hepatic P450s, including control of cholesterol and steroid hormone metabolism and bile acid biosynthesis. However, for certain of these pathways, the exact role of P450s in normal homeostasis is unknown.

There have been major, advances in understanding the functions, genetics and regulation of these enzymes and more recently their structures. However, many fundamental questions about the role of these enzymes in normal homeostasis and in the metabolism and activity of drugs and chemical toxins remain to be answered. The size and diversity of the P450 multi-gene family results in great difficulties in dissecting out the function(s) of individual hepatic P450s, particularly as many of these enzymes involved in foreign compound metabolism exhibit overlapping substrate specificities. In order to understand the functions of this enzyme system an approach is required which will allow the simultaneous inactivation of large numbers of cytochrome P450s.

All cytochrome P450s located in the endoplasmic reticulum receive electrons from a single donor, Cytochrome P450 Reductase (CPR; NADPH:ferrihemoprotein reductase, EC 1.6.2.4); conditional deletion of this protein could therefore inactivate all the P450s located in the endoplasmic reticulum in any particular cell type. It is known from the prior art that P450 expression during development is a vital requirement In recent studies, although a complete deletion of CPR was achieved it was shown to be embryo lethal (Shen et al, J. Biol. Chem. 227, 8, 6536-6541, 2002).

A transgenic non-human animal and/or tissue and/or cells derived therefrom that can be used as a model to study the cytochrome P450-dependent monoxygenase system at a molecular and cellular level and all its associated interactions in drug/toxin metabolism and/or disposition would offer immediate advantages to the art and pharmaceutical, agrochemical, chemical and food industries in general and also permit a further understanding of many biochemical mechanisms.

### Statement of the Invention

According to a first aspect of the invention there is provided use of a non-human transgenic animal comprising cells with a conditional deletion of cytochrome P450 reductase gene in selecting chemical compounds suitable for development as drugs and other products wherein the non-human transgenic animal is selected from the group comprising monkey, dog, cat, rabbit, hamster, rat and mouse.

Reference herein to "product" is intended to include a pharmaceutical, therapeutic, drug, toxin, agrochemical, chemical, food or cosmetic agent/molecule/moiety/ compound/complex or any other natural or synthetic chemical product which may be investigated using the animals or methods of the present invention.

In this specification, except where the context requires otherwise, the words "comprise", "comprises" and "comprising" mean "include", "includes" and "including" respectively.

That is, when the invention is described or defined as comprising specific features, various embodiments of the same invention may also include additional features

According to a further aspect of the invention there is provided use of a non-human transgenic animal comprising cells with a conditional deletion of cytochrome P450 reductase gene in designing chemical compounds suitable for development as drugs and other products wherein the non-human transgenic animal is selected from the group comprising monkey, dog, cat, rabbit, hamster, rat and mouse..

According to a yet further aspect of the invention there is provided use of a non-human transgenic animal comprising cells with a conditional deletion of cytochrome P450 reductase gene in determining tissue disposition of a drug or other chemical compound wherein the non-human transgenic animal is selected from the group comprising monkey, dog, cat, rabbit, hamster, rat and mouse..

According to a yet further aspect of the invention there is provided use of a non-human transgenic animal comprising cells with a conditional deletion of cytochrome P450 reductase gene in toxicity studies wherein the non-human transgenic animal is selected from the group comprising monkey, dog, cat, rabbit, hamster, rat and mouse.

According to a yet further aspect of the invention there is provided use of a non-human transgenic animal comprising cells with a conditional deletion of cytochrome P450 reductase gene in studying the contribution of cytochrome P450s to the etiology of disease states wherein the non-human transgenic animal is selected from the group comprising monkey, dog, cat, rabbit, hamster, rat and mouse.

According to a yet further aspect of the invention there is provided use of a non-human transgenic animal comprising cells with a conditional deletion of cytochrome P450 reductase gene in studying the contribution of cytochrome P450s in pregnancy and/or foetal development wherein the non-human transgenic animal is selected from the group comprising monkey, dog, cat, rabbit, hamster, rat and mouse.

According to a yet further aspect of the invention there is provided use of a non-human transgenic animal comprising cells with a conditional deletion of cytochrome P450 reductase gene in studying the contribution of cytochrome P450s in modulation of hormone function and/or hormone levels and other endogenous pathways in which P450s play a role wherein the non-human transgenic animal is selected from the group comprising monkey, dog, cat, rabbit, hamster, rat and mouse.

According to a yet further aspect of the invention there is provided use of a non-human transgenic animal comprising cells with a conditional deletion of cytochrome P450 reductase gene in studying the contribution of cytochrome P450s in substrate metabolism wherein the non-human transgenic animal is selected from the group comprising monkey, dog, cat, rabbit, hamster, rat and mouse.

According to a yet further aspect of the invention there is provided use of a non-human transgenic animal comprising cells with a conditional deletion of cytochrome P450 reductase gene in identifying products which reduce circulating cholesterol levels wherein the non-human transgenic animal is selected from the group comprising monkey, dog, cat, rabbit, hamster, rat and mouse.

According to a yet further aspect of the invention there is provided use of animal cells having a conditional deletion of cytochrome P450 reductase gene as an *in vitro* screen in product discovery, development and/or disposition/toxicity studies and/or rationalised drug design.

According to a yet further aspect of the invention there is provided use of animal cells having a conditional deletion of cytochrome P450 reductase gene as an *in vitro* screen in studying disease states, pregnancy, foetal development and/or modulation of hormone function and/or hormone levels and or other endogenous pathways and substrate metabolism in which cytochrome P450s play a role.

According to a yet further aspect of the invention there is provided use of animal cells having a floxed cytochrome P450 reductase locus as an *in vitro* screen in product development/discovery and/or disposition/toxicity studies and/or rationalised drug design, in studying disease states, pregnancy or foetal development and/or modulation of hormone function and/or hormone levels and or other endogenous pathways and substrate metabolism in which cytochrome P450s play a role

### Detailed Description of the Invention

Animals suitable for carrying out the present invention are, in general, non-human animals, such as monkeys, dogs, cats, rabbits, rats and mice. Rodent species are preferred, and the mouse is the most preferred species. Animals in every stage of development, including embryonic, neonatal, juvenile, adolescent and adult are included in this description.

Reference herein to deletion of cytochrome P450 reductase (CPR) is intended to include partial or complete ablation, hepatic null or reductase null, "knock-out" or inability to express CPR, the animals having an endogenous CPR gene that can be deleted which may involve removal or all of part of the CPR gene. A reductase null animal may also include an animal where reductase expression is prevented for example by inhibition of CPR mRNA processing.

The term conditional deletion refers to the switching "on" or "off" of a particular gene, in this instance the CPR gene, which is conditional on a specific stimulus.

As previously discussed, hepatic cytochrome P450s play a key role in foreign compound metabolism and normal homeostasis. In order to investigate the functions of this multigene family of proteins we have carried out a conditional deletion of murine hepatic cytochrome P450 reductase. The experimental approach of the present invention has proved effective, resulting in almost complete ablation of hepatic P450 activity, and has generated some unexpected phenotypes. The mice of the present invention produce essentially no bile acids, and exhibit hepatic cholesterol and triglyceride accumulation which, counter-intuitively, is accompanied by a 60-70% reduction in circulating cholesterol and triglycerides.

We have also observed that the mice exhibit a profound reduction in their ability to metabolise drugs and become resistant to toxins activated by the P450 system. In spite of these profound changes, we have unexpectedly found that the animals of the present invention live and are able to reproduce normally, thus demonstrating that hepatic P450-mediated hormone metabolism is not essential for survival or fertility contrary to the teachings of the prior art. Thus, the animal model of the present invention will allow many of the outstanding and fundamental questions about this enzyme system, to be addressed and answered.

Cre is a 38 kDa recombinase protein from bacteriophage P1 which mediates intra and inter molecular site specific combination between loxP sites. The loxP site consists of 13 bp inverted repeats separated by an 8 bp asymmetric spacer region. One molecule of Cre binds per inverted repeat or two Cre molecules line up at one loxP site. The recombination occurs in the asymmetric region. Those 8 bp are also responsible for the directionality of the site. Two loxP sequences in opposite orientation to each other invert the intervening piece of DNA, two sites in direct orientation dictate excision of the intervening DNA between the sites leaving one loxP site behind so as to precisely remove the DNA.

The animals of the present invention may preferably be produced by a method comprising the steps of transfecting embryonic stem cells with a CPR targeting construct, selecting animals homozygous for a floxed CPR locus (CPR ^{lox/lox}) and crossing said animals with a line where Cre expression is regulated.

Reference herein to regulation of Cre expression is intended to include constitutive and inducible regulation by either an endogenous or exogenous agent or stimulus.

Preferably, Cre expression is regulated by a promoter for example a rat albumin promoter or CMV promoter or CYP1A1 promoter.

It will be appreciated that in the instance of using a rat albumin promoter as the albumin promoter becomes active neonatally CPR expression and activity decreases to an extent where at 6 to 8 weeks of age there is no immunoreactive band corresponding to CPR. In this manner CPR expression is conditionally deleted by aging and remains deleted as a consequence of the continuous activity of the albumin promoter.

It will also be appreciated that site specific recombinase methods other than cre-recombinase will also be applicable to a method of producing the animals of the invention, for example using Flp/ftr. The Flp recombinase is a 45kD protein encoded by the 2Mm plasmid of the *yeast Saccharomyces cerevisiae.* It would also be possible to use a C31/attB, attP or a gene trap vector.

The invention also discloses a CPR targeting construct covering exons 3 to 16 of a CPR gene.

Preferably, the construct comprises a 12 kb *SalI* fragment.

Preferably, the construct is included in a cassette, flanked by same-orientation *loxP* sites and optionally containing a selectable marker for example neomycin.

Preferably, the cassette also includes a promoter for example a herpes simplex thymidine kinase (*hsv-tk*) promotor.

Preferably, the casette is inserted into intron 4.

According to a yet further aspect of the invention there is provide use of the construct of the present invention in the production of an animal according to the present invention.

We have been able to generate mice where CPR is deleted in the post-natal period in any tissue, using by way of example only the Cre/loxP system (Le and Sauer, Mol. Biotechnol., 17, 269-275, 2001). Hereinafter the intriguing phenotypes observed with a hepatic deletion of the enzyme system will be described.

It will be appreciated that other methods of deleting or ablating CPR or reducing/preventing its expression may also be applicable with the method of the present invention. For example such methods include, siRNA technology (small inhibitor RNA technology). siRNA refers to the introduction of homologous double stranded RNA (dsRNA) to specifically target a gene's product, resulting in null or heterozygote phenotypes.

It will be appreciated that the hepatic nulls or reductase null heterozygote animals of the present invention may in one aspect of the invention be used as an *in vivo* screen to determine any one or more of the following parameters:
(i) role of cytochrome P450s or cytochrome P450 reductase in the disposition of an organic, low molecular weight small molecule, preferably <1000Da. These studies can be carried out by the oral, dietary or intravenous administration of the compound to be tested and evaluation of the rates of elimination in the bile, urine or faeces or alternatively, by measuring circulatory levels of the compound. In addition, the level of metabolism or the disposition of a drug/compound *in vivo* could be determined by measuring the concentration of the drug/compound at different time points detected in different tissues, e.g. by using radio-labelled compound followed by whole body autoradiography or by analysis of drug/compound levels in a particular tissue e.g. using analytical methods such as mass spectrometry;
(ii) pathways of drug/product disposition. Deletion of P450 reductase in extrahepatic cells will allow the role of metabolism in drug uptake and intracellular drug concentration to be determined through pathways of drug disposition. By comparing the metabolites produced in control or P450 hepatic reductase null animals it will be possible to establish which metabolites are produced as a direct consequence of the activity of the hepatic cytochrome P450 system;
(iii) routes of Phase II drug/product disposition of a parent compound. In the absence of hepatic cytochrome P450 activity it will be possible to establish whether other enzymes are involved in the disposition of the parent compound. This may not be immediately obvious in the control animals, where the situation is complicated by P450-mediated reactions;
(iv) role of drug transporters in drug uptake across for example, the gastrointestinal tract and, into the liver. In the absence of cytochrome P450 expression in tissues such as the liver or the GI tract the evaluation of the role of other enzymes or proteins in determining drug bioavailability, in particular the role of drug transporters will be facilitated. At the present time, most of the inhibitors of drug transporters are also inhibitors of the cytochrome P450 system. The use of such compounds *in vivo* to study their effects on drug bioavailability or metabolism does not allow the investigater to distinguish one pathway from another. However, in the absence of P450 activity there is greater confidence that the inhibitors are now specific for the drug transporters and therefore studies on the role of drug transporters in determining bioavailability will become feasible using the animals of the present invention;
(v) role of cytochrome P450 reductase in pathways of chemical toxicity. The cytochrome P450 system can detoxify drugs and foreign compounds through metabolism, e.g, in the case of many anticancer drugs and it can also catalyse the conversion of many compounds to products which are chemically reactive and toxic. The conditional reductase null model of the present invention may be used to evaluate the role of the P450 system in these reactions. Identification of a role for P450s in these reactions will advantageously permit studies to be carried out to evaluate whether such reactions are relevant to man and also will facilitate the redesign of the molecules to avoid these unwanted effects;
(*vi*) role of the P450 system in normal metabolic processes/homeostasis. In addition to its role in drug disposition the cytochrome P450- dependent mono-oxygenase system carries out a large number of important metabolic functions, some of the consequences of these reactions are unknown. The P450 reductase animal model of the present invention may advantageously be used to delete cytochrome P450 function in a tissue- or cell- specific manner to establish the role of particular P450 pathways in normal homeostasis. This will allow an evaluation of whether these particular pathways, for example and without limitation, retinoic acid metabolism in the skin or neurosteroid biosynthesis in the brain, provide novel targets for the development of new drugs. In addition, the potential ablation of cytochrome P450 activity will allow novel disease models to be generated, for example and without limitation, deletion of the Vitamin D1,25-hydroxylase activity in the liver and kidney will generate a Vitamin D-deficient mouse, deletion of steroid hormone biosynthesis in the adrenal will generate a mouse deficient in steroid hormones. Therefore, in one embodiment of the invention the P450 reductase null animal may be used to generate disease models for the potential testing of new drugs which may be therapeutic against the phenotypes observed;
(vii) role of the P450 system in the pathogenesis of disease. Because of the role of the cytochrome P450 system in normal homeostasis, the P450 reductase null mouse model may be used to evaluate the role of the P450 system in disease aetiology and progression. This would find particular utility in degenerative diseases where the cytochrome P450 system is known to have the capacity to generate chemically reactive oxygen species that may potentiate degenerative processes.

It will be appreciated that the hepatic nulls or reductase null heterozygote animals of the present invention may also in one aspect of the invention be used as an *in vivo* screen to investigate any one or more of the following events or parameters:
(i) pharmacological potency of agents subject to first pass metabolism, i.e. which would otherwise have been metabolised. With some drugs in development it is not possible to establish their *in vivo* pharmacological activity because high rates of first-pass cytochrome P450 mediated metabolism do not allow sufficient concentrations to be obtained to measure a pharmacological effect. This may result in valuable and potent compounds not being developed. However, in the hepatic reductase null mice of the present invention, for such compounds high drug levels may be obtainable and their pharmacological potency evaluated. In the event that the compound demonstrates *in vivo* potency the compound could subsequently be modified to circumvent the high rates of P450 metabolism;
(ii) occurrence and rate of extrahepatic metabolism in for example and tissues such as and without limitation kidney, brain, GI tract, skin and/or adrenal gland. It has been estimated that up to 30% of drug metabolism may occur in tissues other than the liver and may well vary according to the specific drug involved. The rate of extra hepatic drug metabolism may be an important parameter in drug disposition/toxicity as well as in altering susceptibility to events such as drug-drug interactions. The hepatic P450 reductase null mouse of the present invention could therefore be used to establish whether any particular drug or chemical entity is metabolised in extrahepatic tissues and what that rate of metabolism is. Extrahepatic metabolism may also be an important parameter for the activation of pro-drugs in particular cell types in order for them to exert their therapeutic activity, accordingly the animals of the present invention may find utility in pro-drug activation studies ;
(iii) rate of hepatic metabolic clearance as a determinant in product/drug distribution and pharmacokinetics. Establishing the rate of metabolic clearance is a key pharmacokinetic parameter which determines the biological half life and activity of therapeutic agents and foreign chemicals. In the absence of P450 metabolism the role of these enzymes in metabolic clearance can be clearly established and will allow a better understanding of how potential drugs or compounds may be handled in man;
(iv) facilitate in lead product/compound selection based on *in vivo* metabolic parameters. Based on metabolic parameters, the hepatic P450 reductase null mouse of the present invention may be used as a tool in the rapid screening of large numbers of chemical entities to establish which have the most appropriate pharmacokinetic parameters for use in man. It would also identify compounds which require chemical modification in order to optimise their pharmacokinetic properties;
(v) cross into drug discovery mouse models so as to establish the effects of metabolism on pharmacological activity and therapeutic outcome. It is known from the prior art that an increasing number of transgenic reporter mice are being developed for pre-clinical drug development, toxicology and drug discovery purposes. In one embodiment of the invention the hepatic reductase null model may be advantageously crossed into one of these reporter mouse systems, thereby allowing a much clearer evaluation of the role of metabolism on the pharmacological or toxicological effects of a compound under investigation;
(vi) assessment of relevance and occurrence of pharmacologically active metabolites. Many products/drugs first require metabolism by the cytochrome P450 system to generate pharmacologically active products. It is also important to evaluate whether the metabolism of a potential drug or product results in inactivation or whether the product retains or even has enhanced pharmacological or toxicological properties. Comparison of pharmacological effects in animals which do or do not contain P450 metabolism will allow an evaluation of pro-drug or chemical activation to be established;
(vii) distinguish between toxicity due to product metabolic activation or other mechanisms. Many chemicals agents are toxic as a consequence of their conversion to reactive metabolites by the cytochrome P450 system. In the event that a drug or product in development exerts an idiosyncratic toxicity *in vivo* the conditional P450 reductase null mouse may be used in one embodiment of the invention to evaluate whether metabolic activation by the cytochrome P450 system is involved. If this is the case, the nature of the toxic metabolites can be established and the molecule redesigned to avoid such potentially toxic events;
(viii) establish role of P450 or P450 reductase as a rate limiting step in drug/compound disposition. In addition to using conditional deletion of reductase in a particular cell type experiments may be carried out in mice heterozygous for P450 reductase expression, i.e. mice are bred which contain one P450 reductase null allele and a functional P450 reductase allele. Under these circumstances, P450 activity would be predicted to be reduced as a consequence of a limited rate of electron flow to the P450 enzymes. Evidence that this is the case has already been obtained, therefore this particular model of the invention may be used to establish the role of the P450 system in the parameters described above. In the event that the metabolism of a drug is not altered in the P450 reductase heterozygous mice, this will indicate that electron transfer to the P450 system is not an important determinant in the rate of drug disposition;
(ix) role of bile flow on drug elimination. The hepatic P450 reductase null mice of the present invention have been shown to have compromised bile acid production. The animals of the present invention may therefore be used to establish the effects of bile flow on the rate and pathways of drug disposition. Such studies may be particularly valuable for compounds which cause choleostasis; or
(x) study drug/drug interactions due either to P450 or drug transporter effects. As described above, if hepatic P450 activity is absent it would be possible to carry out drug-drug interaction studies in order to evaluate whether such effects are due to interaction with the cytochrome P450 system or with other pathways of drug uptake and disposition, such as with the drug transporters. Such information is important both for drug regulatory purposes and for identifying potential difficulties in the use of a drug in the clinic.

It will be appreciated that the hepatic nulls or reductase null heterozygote animals of the present invention may also in one aspect of the invention be used in any one or more of the additional following situations:
(i) as a cross into a disease model so as to establish the role of compound metabolism in pharmacological/toxicological effects of the product;
(ii) as a cross into a multi drug response protein (Mdr1 and Mdr2) null mouse so as to evaluate a dual role of both P450s and Mdr in product availability studies;
(iii) as a cross into reporter animals, for example and without limitation oxidative stress sensitive reporters for the study of degenerative diseases. Crossing the conditional reductase nulls of the present invention into mice carrying reporters for specific diseases will allow the role of the P450 system in the aetiology and progression of that disease to be evaluated. The reductase null mice can also be crossed into reporter mice, e.g. which reflect particular types of cellular stress responses so that the role of the P450 system in metabolic activation of a chemical and mechanism of the toxic effects can be established more easily. In addition, many drugs and foreign compounds have the capacity to induce or suppress the cytochrome P450 system. In many cases whether this effect is due to the parent compound or to one of its metabolites is unknown. This is particularly the case e.g. for chemopreventive agents. In one example of this embodiment of the invention, a reporter system may be used, e.g. where the promoter of a gene is linked to a reporter such as *LacZ*, alternatively in a toxicology screen the promoter could be activated by a chemical as in the case of the CYP1A1-*LacZ* system;
(iv) in generating cell lines from the animals of the present invention, with no P450 reductase activity, e.g. fibroblasts or by introducing an immortalising gene, e.g. SV40 T antigen, for any cell type. In addition to the *in vivo* experiments, all the experiments described above could be carried out *in vitro.* These could be carried out either using primary cells derived from the conditional hepatic reductase null mice or from cell lines derived from them, e.g. by using fibroblast cell lines or by introducing an immortalising gene into the cells, either by crossing the reductase mice into mice carrying an immortalising gene such as SV40 large T antigen or by introduction of the immortalising gene to the primary cells in culture; or
(v) in generating novel *in vivo* mutation tests so as to evaluate the role of P450/P450 reductase in chemical mutagenesis. An important aspect of chemical and drug development is to establish whether the chemical entity has toxic or particularly mutagenic potential. *In vivo,* the P450 reductase null mice could be crossed into mice which report mutagenic events such as big blue mice to establish the role of the P450 system in chemical mutagenicity. Alternatively, *in vitro* studies could be carried out using hepatic sub-cellular fractions from control or reductase null animals to establish the role of P450 metabolism in any mutagenic effect observed, e.g. using S9 fractions in the Ames test.

The cytochrome P450-dependent monooxygenase system is distributed throughout the body and different enzymes are expressed in a tissue- and cell-specific manner. In one embodiment of the invention the Cre recombinase system is specifically targeted in a conditional way to particular cell types to enable the specific deletion of particular enzymes in those cells. This may be achieved preferably by the use of the promoters of cytochrome P450 genes or promoters of other drug metabolising enzymes to drive Cre recombinase so as to direct expression to cells containing these enzymes. In addition, the promoters of transcription factors which mediate the regulation of cytochrome P450 genes may preferably be used as a mechanism of delivering Cre specifically to cells where particular P450 enzymes are expressed.

According to a yet further aspect of the invention there is provided an animal or an animal cell comprising a Cre recombinase system and a promoter for driving expression of Cre recombinase in a tissue or cell specific manner, the promoter being selected from the group comprising a cytochrome P450 promoter, a promoter of another drug metabolising enzyme or a transcriptional factor promoter.

Cytochrome P450 CYP1A1 is a highly regulatable gene whose expression is determined by the presence of xenobiotic responsive elements contained within the promoter. We have previously demonstrated that this promoter provides an extremely tight on/off switch for regulating gene expression in response to exogenous chemical agents i.e. constitutive levels of cytochrome P450 1A1 are extremely low but are highly inducible on exposure of animals or cells to a range of specific chemical entities. CYP1A1 is regulatable in virtually all cell types expressing the cytochrome P450 system, accordingly in one embodiment of the invention administration of an exogenous agent causes deletion of the P450 system in virtually all relevant cells of the animal. Therefore, crossing P450 reductase floxed mice with CYP1A1 Cre mice will allow the conditional regulation of Cre in many cell types. In other embodiments of the invention, a transcriptional factor promoter such as the pregnane x-receptor (PXR) promoter is used to generate tissue-specific conditional deletions. This receptor is predominantly expressed in liver, intestine and kidney and therefore the promoter using this to drive Cre recombinase would allow specific deletion of the P450 system in these tissues. A further alternative is the promoter of the transcription factor, Nrf2, which again is regulated by a distinct class of compounds such chemopreventive agents including coumarin, ethoxyquin and butylated hydroxyanisole. A yet further example is the cytochrome P450 CYP2B1 or CYP2B6 promoter which would provide constitutive expression in the alveolar type 2 cells and clara cells of the lung as well as regulatable expression specifically in the liver by a range of compounds including barbiturate drugs. Additionally, the use of the cytochrome P450 CYP3A or CYP4A promoters would allow regulation of Cre in the liver or intestine by compounds such as dexamethasone or clofibric acid respectively. Accordingly, in these particular embodiments of the invention a promoter such as hereinbefore described is used for tissue/cell specific Cre expression in a constitutively or inducible manner so that specific tissue/cells may comprise a CPR gene deletion.

In one embodiment of the invention, the reductase null animals of the present invention may advantageously be used to delete the P450 system in a tissue or cell specific manner. Such animals may be used for the applications hereinbefore described, where the role of the P450 system in product/drug metabolism, detoxification, normal homeostasis or in disease etiology is to be studied. It is envisaged that this embodiment will also allow other effects, such as drug transporter- mediated effects, to be studied in those tissues or cells in the absence of metabolism.

Preferably, tissue specific deletions are achieved by crossing CPR-floxed mice into mice where cre recombinase is driven off a tissue- or cell-specific promoter, for example that of an intestinal fatty acid binding protein, so as to obtain a GI tract specific deletion or in another example, bovine keratin VI, so as to obtain a skin epithelial cell deletion.

As previously mentioned, Cre regulation may be constitutive or inducible. In another embodiment of the invention, the regulation of cre may be conditional, that is to say its expression can be regulated by the administration of an exogenous agent, for example and without limitation by using the tamoxifen switch system, or the tetracycline - inducible expression system. One such example is where cre is driven off the CYP1A1 promoter which is regulated by a large number of exogenous foreign compounds. Cre recombinase activity is silent until the exogenous inducing agent is applied, i.e. there is no phenotype observed until this is done. Accordingly, it is envisaged, in an *in vivo* experiment this would advantageously allow the same animal to be used as both test and control. The CYP1A1 system may be used to obtain a liver predominant deletion or a deletion in adult or foetal tissue where the CYP1A1 promoter is active. The tissue/cell specific or the pan-deletion may be used in product/drug development, disposition and toxicity studies as hereinbefore described.

The cytochrome P450 system, in addition to its role in drug and foreign compound metabolism, plays an important role in the biosynthesis and metabolism of a range of hormones and signalling molecules in a variety of different tissues. For example, in steroid hormone biosynthesis (estrogens, prostacyclins, androgens, glucocorticoids) in the gonads and adrenal glands, arachadonic acid metabolism in the kidney, thromboxane in platelets, prostaglandin metabolism in the lung and prostate gland, Vitamin D3 metabolism in the liver and kidney, neurosteroid biosynthesis/metabolism in the brain and hypocampus, catechol estrogen biosynthesis in adrenal glands, foetal liver and hypothalamus, bile acid biosynthesis in the liver, fatty acid metabolism in the liver and lungs and retinoic acid metabolism in the foetus, liver and skin. In addition P450s play an important role in pregnancy and foetal development therefore the present invention provides a tool with which to study their role in these conditions.

Accordingly the animals of the present invention, in a further aspect of the invention may be used to modulate the functions and the levels of associated hormones in any of the above cell types to generate a disease model or a model for product/drug discovery or a model to verify or assess functions of the P450 system.

We have observed that deletion of hepatic P450 reductase resulted in almost complete ablation of bile acid production the major route of cholesterol disposition, and that this was accompanied by a significant hepatic accumulation of cholesterol. Surprisingly and contrary to expectation however it was also accompanied by a reduction in circulating cholesterol levels, thus identifying a hitherto unidentified role for the P450 system in controlling both hepatic and circulating cholesterol levels. With these observations in mind, in a yet further aspect of the invention there is provided use of the animals of the present invention in assessing the role of bile acids in disease states and in drug disposition studies, as a model for studying choleostasis, assessing the role of cholesterol in atherogenesis and other disease states.

According to a yet further aspect of the invention there is provided use of the animals of the present invention in identifying novel drugs which inhibit the P450 system and which may also be used to reduce circulating cholesterol levels.

According to a yet further aspect of the present invention there is provided use of the animal tissues and/or cells derived therefrom of the present invention as an *in vitro* assay for the study of any one or more of the following events/parameters:
(i) role of transporters in product uptake and efflux;
(ii) identification of product metabolites produced by the cytochrome P450 system;
(iii) evaluate whether candidate products are P450 substrates;
(iv) assess drug/drug interactions due to P450 effects;
(v) cross into Mdr1 and/or Mdr 2 null mice so as to evaluate a dual role of P450s and Mdr or;
(vi) use of an S9 fraction from null mice in an Ames test so as to determine potential P450-mediated chemical mutagenicity.

Reference herein to animal tissues and/or cells derived therefrom is intended to include primary hepatocytes, hepatic cell lines, hepatic microsomal fractions, liver slices, and/or any other tissue, for example and without limitation, kidney, lung or brain tissue and/or cells, for example and with out limitation, keratinocytes or chondrocytes in which, under normal conditions cytochrome P450 and/or cytochrome P450 reductase is expressed and/or transported thereto or therefrom. The tissue and/or cells of the present invention being derived from the animals of the present invention or manufactured *in vitro* or *ex vivo,* all of which are encompassed within the scope of the present invention.

It will be appreciated that tissue and/or cells derived from the hepatic nulls or reductase null heterozygote animals of the present invention may also in one aspect of the invention be used as a tool/screen to investigate any one or more of the following events or parameters:
(i) establish involvement of the cytochrome P450 system in hepatotoxicity/carcinogenicity;
(ii) establish whether hepatic product metabolism is involved in toxicity to other organs;
(iii) establish whether hepatic production of a product metabolite (i.e. pro-drug activation) is important for pharmacological action/toxicity of a given candidate compound;
(iv) establish whether a parent product or its P450 generated metabolite(s) mediate induction of drug metabolising enzymes, liver growth or other phenotypic effects;
(v) investigate role of the P450 system in peroxisome proliferation;
(vi) investigate role of cytochrome P450 reductase in product disposition, toxicity or metabolic activation or;
(vii) investigate presence of secondary sites of toxicity in the absence of toxicity due to P450 mediated compound metabolism.

According to a yet further aspect of the invention there is provided a transgenic animal, tissues and/or cells derived therefrom as hereinbefore described, modified to contain or express DNA encoding a human a cytochrome P450 DNA.

Described but not claimed is the use of a transgenic animal, tissues and/or cells derived therefrom that has been modified to contain and express DNA encoding a human DNA cytochrome P450 so as to establish the role of human P450s in foreign or endogenous compound metabolism, toxicity or normal homeostasis.

Described but not claimed is the use human cells, especially hepatocytes introduced into an immune-deprived reductase null animal of the present invention so as to investigate contribution of human cells or hepatocytes in P450-mediated product metabolism and/or toxicity.

Human cells or hepatocytes may grow in, for example the liver or spleen of SCID (severe combined immune deficiency) mice. SCID mice are homozygous for the *Prkdc^{scid}* mutation and lack both T and B cells due to a defect in V(D)J recombination. Therefore, these mice easily accept foreign tissue transplants, including human tumors, making them effective models for testing new cancer treatments and as hosts for human immune system tissues (i.e., SCID-hu).

Thus, only the human cells or hepatocytes will contribute to cell or hepatocyte-mediated P450 metabolism and thus may be advantageously used as a human metabolism model in the absence of background mouse metabolism.

Described but not claimed is the use of a transgenic animal, tissues and/or cells derived therefrom as hereinbefore described that have been modified to contain and express DNA encoding a human DNA protein so as to establish the role of said protein in a P450 metabolism free environment.

Other human proteins of interest, for example and without limitation, drug transporters, may be introduced onto the

Mdr/reductase null background of the animal or animal cells of the present invention and thus advantageously permit the functions of the human Mdr proteins to be studied on a P450 metabolism free or murine Mdr null background.

It will be appreciated that the present invention provides a tool for the investigation of disease states such as choleastasis, artherogenesis, hormonal unbalances, neurological disorders, degenerative diseases, skin conditions, cardiovascular disease, cancer and glaucoma and any other disease in which P450s play a role.

The invention will now be described by way of example only with reference to the following figures wherein:
Figure 1 illustrates targeting of the mouse CPR gene;
Figure 2 illustrates liver-specific deletion of CPR in hepatic CPR null mice;
Figure 3 illustrates characterisation of hepatic CPR null phenotype;
Figure 4 illustrates CPR and P450 expression in hepatic CPR null mice;
Figure 5 illustrates drug treatment of hepatic CPR null mice;
Figure 6 illustrates the pshuttle-CMV-3A4, full length of human CYP3A4 cDNA fusion with human P450 reductase cDNA (codons for first 57 amino acids deleted) ligated into the Kpn I/Not I site of the shuttle vector pshuttle-CMV;
Figure 7 illustrates 1' hydroxy-midazolam production by mouse hepatocyes during midazolam metabolism;
Figure 8 illustrates midazolam concentrations during midazolam metabolism by mouse hepatocyes;
Figure 9 illustrates 1' hydroxy-bufuralol production by mouse hepatocyes during bufuralol metabolism and;
Figure 10 illustrates bufuralol concentrations during bufuralol metabolism by mouse hepatocyes;

### Detailed Description of the Figures

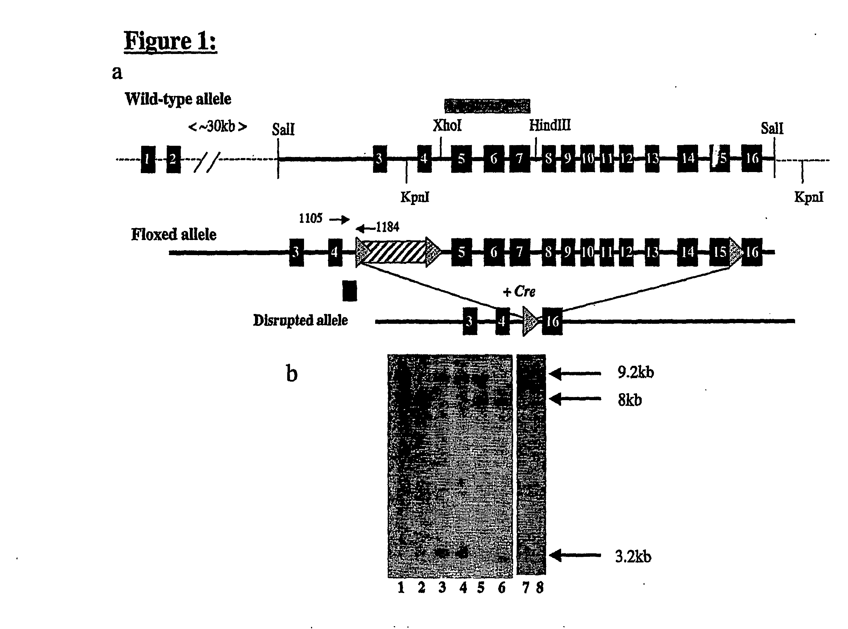

**Figure 1****:** Targeting of the mouse CPR gene.
   **a** Maps of the wild-type, floxed and disrupted CPR alleles. A 12 kb *SalI* fragment, containing exons 3-16 of the mouse CPR gene was cloned and used for gene targeting. Exon 1 (untranslated) and exon 2 reside approximately 30kb upstream, and are shown only for information. *LoxP* sites are indicated by triangles, and the selectable marker (*hsv-tk-neo*) is indicated by the hatched box in intron 4.
   **b** Southern analysis of tail DNA from CPR^{+/+}, CPR^{+/-} , CPR^{lox/+}, CPR^{lox/-} and CPR^{lox/lox} mice. Genomic DNA was digested with *KpnI* and hybridised with a 1.5 kb *XhoI-HindIII* fragment as shown in a. The wild-type allele (CPR⁺) is represented by a fragment of 8kb, the targeted allele (CPR^{lox}, which also contains the selectable marker cassette), by a fragment of approximately 9.2kb, whilst the deleted allele (CPR⁻) is represented by a fragment size of 3.2kb. Lanes 1,5 = CPR^{lox/+}; Lanes 3,4 = CPR^{lox/-}; Lane 2 = CPR^{+/+}; Lane 6 = CPR^{+/} ; Lanes 7,8 = CPR^{lox/lox};

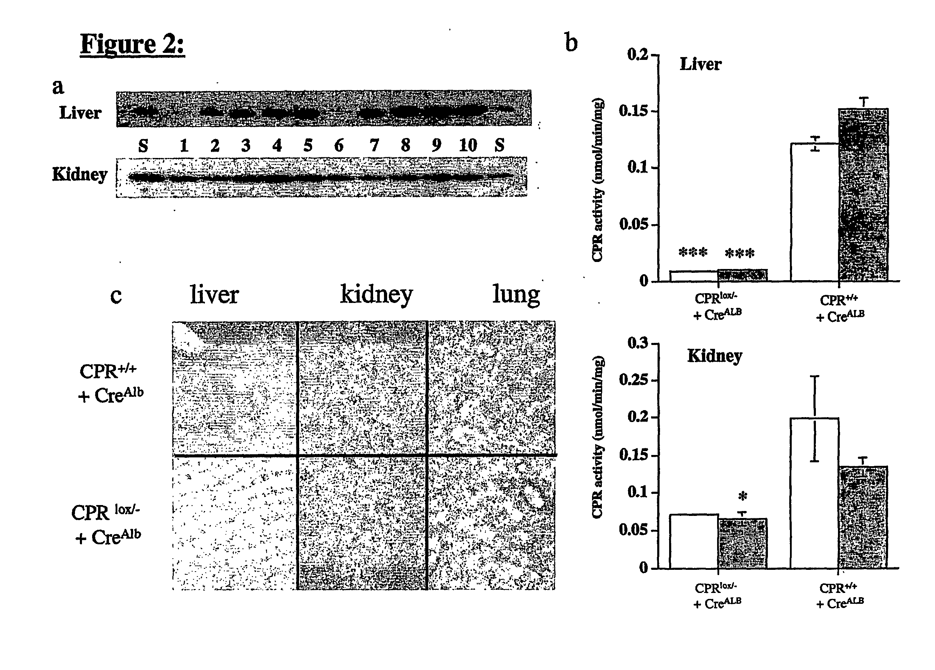

**Figure 2****:** Liver-specific deletion of CPR in hepatic CPR null mice
   **a** Representative immunoblot showing CPR protein levels in liver and kidney of the following mice- lanes 1,6 = CPR^{lox/-} + Cre^{ALB} male, female; lanes 2,7 = CPR^{lox/-} male, female; lanes 3,8 = CPR^{lox/+} + Cre^{ALB} male, female; lanes 4,9 = CPR^{lox/+} male, female; lanes 5,10 = CPR^{+/+} + Cre^{ALB} male, female; S = CPR standard
   **b** Hepatic and renal CPR activity in male (white bar) and female (grey bar) wild-type (CPR^{+/+} + Cre^{ALB}) and hepatic CPR null mice (CPR^{lox/-} + Cre^{ALB}), (n=3). Values are expressed nmol cytochrome c reduced/min/mg microsomal protein ±SBM.
      * p< 0.05, *** p<0.001 using an unpaired t-test.
   **c** Immunostaining of liver, kidney and lung sections from wild-type (CPR^{+/+} + Cre^{ALB}) and hepatic CPR null mice (CPR^{lox/-} + Cre^{ALB}) with anti-CPR antiserum. Data shown is representative of that found in each group (n=3).

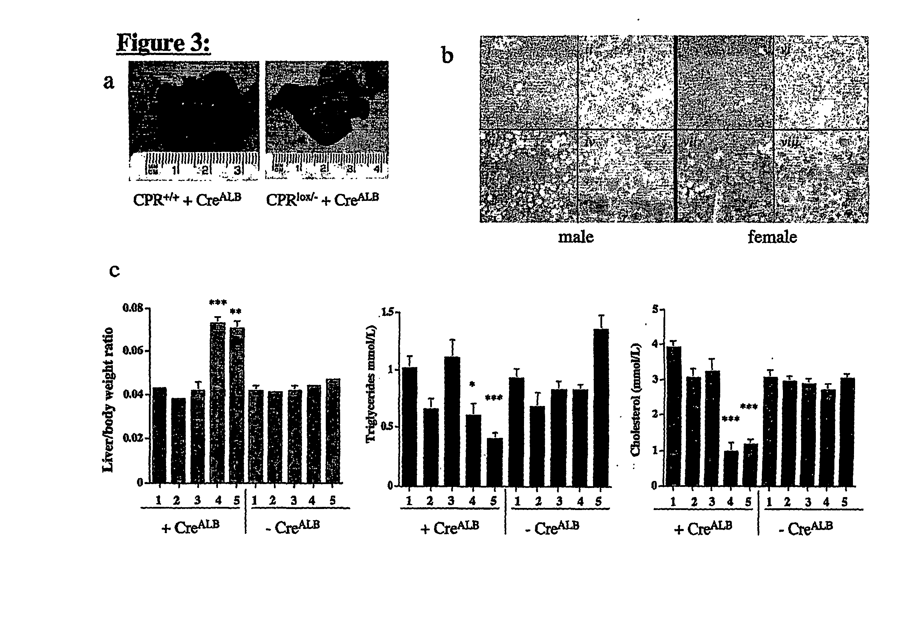

**Figure 3****:** Characterisation of hepatic CPR null phenotype
   **a** Photographs show typically enlarged size and pale appearance of liver from hepatic CPR null mouse (right) and wild-type mouse (left).
   **b** Representative sections from livers of male (left) and female (right) CPR^{+/+} + Cre^{ALB} (top) (control) and CPR^{lox/-} + Cre^{ALB} CDR null, (bottom) mice, stained with haematoxylin and eosin (i, iii, v, vii) or Oil Red O (ii, iv, vi, viii).
   **c** Liver/body weight ratio, serum triglycerides and cholesterol in adult hepatic CPR null and wild-type mice (n = 4-12). Values are expressed as mean ± SEM. Lanes 1= CPR^{+/+}; 2 = CPR^{lox+}; 3 = CPR^{+/-} ; 4 = CPR^{lox/-}; 5 = CPR^{lox/lox}.
      * p< 0.05, ** p<0.005, *** p<0.001 using an unpaired t-test.

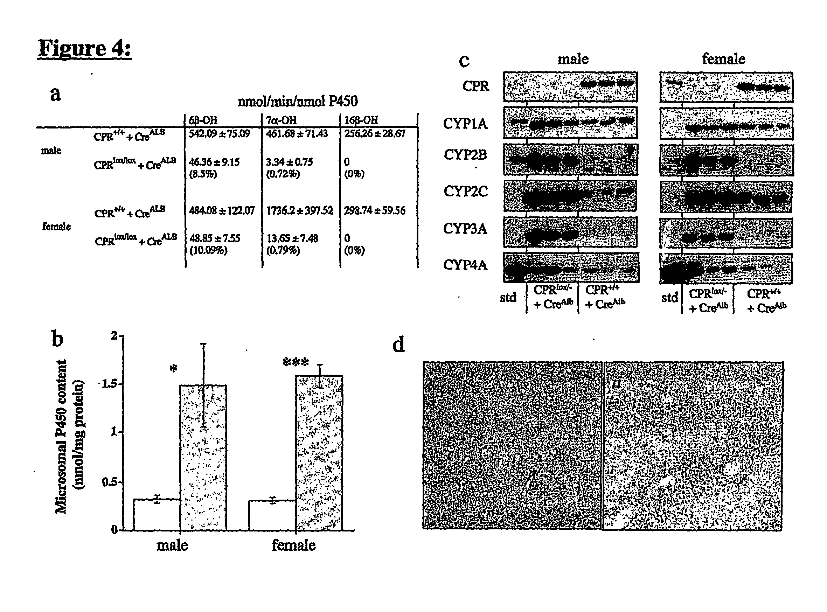

Figure 4: CPR and P450 expression in hepatic CPR null mice
   **a** Hepatic testosterone 6β-hydroxylase activity in adult male and female CPR^{+/+} + Cre^{ALB} (white bars) and CPR^{lox/-} + Cre^{ALB} (grey bars) mice (n=3). Values are expressed as mean ± SEM.
      p< 0.05 using an unpaired t-test.
   **b** Testosterone 6β, 7α and 16β hydroxylation in liver microsomes from CPR^{+/+} + Cre^{ALB} and CPR^{lox/lox} + Cre^{ALB} mice. Activities expressed in pmol/min/nmol P450, mean ± SEM, figures in parentheses represent % activity in samples relative to wild-type =100%. Assay carried out in duplicate, n=4;
   **c** Immunoblotting of hepatic microsomes from adult male and female CPR^{+/+} + Cre^{ALB} and CPR^{lox/-}+ Cre^{ALB} mice with polyclonal antisera, to CPR and various P450s. std = protein standard.
   **d** Immunostaining of liver sections from female CPR^{lox/-} + Cre^{ALB} (i) and CPR^{+/+} + Cre^{ALB} (ii) mice with polyclonal antisera to CYP3A1, phase contrast, magnification x4.

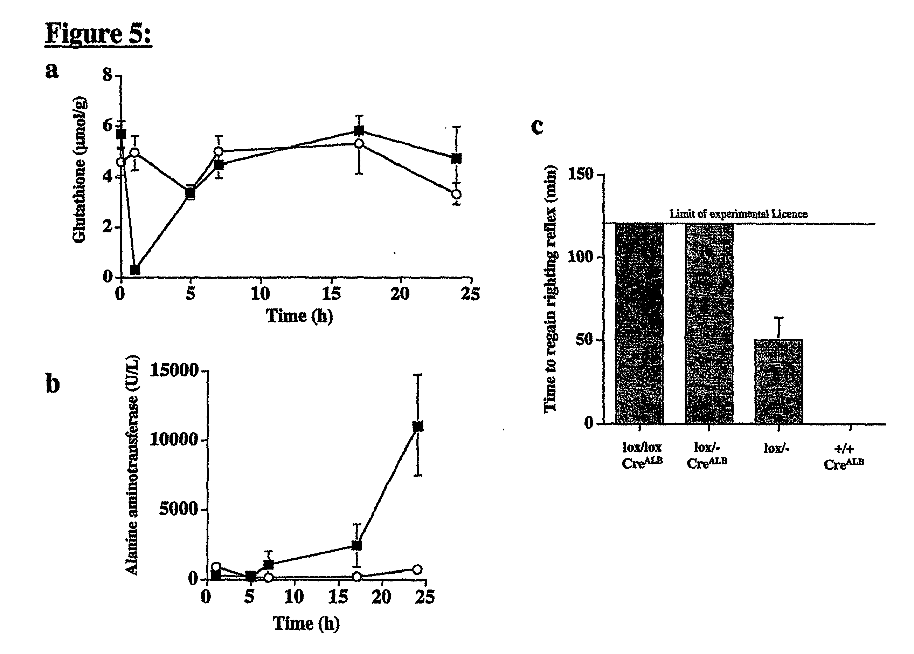

**Figure 5****:** Drug treatment of hepatic CPR null mice
   **a** Hepatic glutathione and **b** serum alanine aminotransferase levels in adult male CPR^{+/+} + Cre^{ALB} (closed squares) and CPR^{lox-}+ Cre^{ALB} (open circles) mice following treatment with acetaminophen at 300mg/kg i.p. at time zero (n=3). Values are expressed as mean ± SEM.
   **c** Time taken for regain of righting reflex in adult male mice of various genotypes following treatment with sodium pentobarbital at 20 mg/kg i.p. (n=3). Values are expressed as mean ± SEM.

### Methods

### Generation of CPR floxed and knockout mice

A replacement-type targeting vector was constructed from a 12 kb *SalI* fragment containing exons 3-16 of the mouse CPR gene. A cassette, flanked by same-orientation *loxP* sites and containing a selectable marker (neomycin (*neo*))*,* driven by the herpes simplex thymidine kinase (*hsv-tk*) promotor, was inserted into intron 4. A third *loxP* site was cloned into intron 15. The construct was transfected into GK129/1 embryonic (ES) cells by electroporation and the ES cells were subsequently plated out into 96 well plates and G418 selection applied. Eight G418-resistant clones were found to have undergone specific homologous recombination as demonstrated by Southern analysis and two of these correctly targeted ES cell clones (CPR^{lox/+}) were expanded, injected into C57BL/6 blastocysts and transferred into 2.5 days post-coitum (dpc) recipient pseudopregnant mice. Male chimerae were bred to C57BL/6 mice and heterozygous offspring were screened by Southern analysis to confirm germline transmission of the CPR^{lox/+} genotype. Five of these ES clones containing the CPR^{lox} locus were transiently transfected with a vector containing the Cre recombinase gene (pMC1Cre). Colonies were obtained without selection and were isolated into a 96 well plate; after 3-4 days the plates were split into two duplicate plates, and after a further 3-4 days one plate was frozen and the other further split. G418 selection was applied for up to 5 days to one of these plates in order to identify sensitive colonies. DNA was prepared from the duplicate plate after 3-4 days later. ES clones (800) were tested by Southern blot analysis, using a 600 bp PCR fragment with primers 1105 and 1184 (Fig. 1a). Nine of those clones screened showed excision of the floxed sequence including exons 5 to 15. Two of these ES cell clones (CPR^{+/-}) were expanded and chimaeric mice generated. Male chimerae were bred to C57BL/6 mice and heterozygous offspring were screened by Southern blot analysis to confirm germline transmission.

### Mouse breeding and maintenance

CPR^{+/-} mice were maintained by random breeding with CPR^{+/+} mice on a 129P2xC57BL/6 genetic background. CPR^{+/-} mice were crossed with each other to determine the viability of a CPR^{-/-} genotype.

CPR^{lox/+} mice were crossed to produce homozygous CPR^{lox/lox} mice and maintained by random breeding on a 129P2xC57BL/6 genetic background. The CPR^{lox/lox} line was bred with CP^{+/-} to generate a CPR^{lox/-} line.

A transgenic mouse line expressing Cre recombinase under control of the rat albumin promotor (4) was crossed onto both the CPR CPR^{lox/lox} (CPR^{lox/lox} + Cre^{ALB}) and CPR^{lox/-} (CPR^{lox/-} + Cre^{ALB}) lines to generate liver-specific CPR conditional knockout mice. The presence of the Cre^{ALB} transgene was determined by PCR (data not shown).

### Drug treatment of mice

Pentobarbital sleep time - adult male CPR^{lox/lox} + Cre^{ALB}, CPR^{lox/-} + Cre^{ALB}, CPR^{lox/-}, and CPR^{+/+} + Cre^{ALB} mice (n=3) were given a single intraperitoneal (i.p.) dose of sodium pentobarbitone (Sagatal) at 20 mg/kg body weight. The time taken for the mice to lose, and subsequently to regain, their righting reflex was measured. Acetaminophen treatment - adult male CPR^{lox/-} + Cre^{ALB} and CPR^{+/+} + Cre^{ALB} mice were administered acetaminophen intraperitoneally at 300 mg/kg body weight in phosphate-buffered saline (PBS). At various time-points after treatment, mice were sacrificed (n=4) and blood and tissues taken for analysis.

### Immunoblotting and biochemical assays

Microsomal fractions were prepared from frozen tissues by differential centrifugation and protein concentration determined as previously described. Western blots were carried out as described previously using 9% sodium dodecylsulphate (SDS/PAGE) gels and electroblotted onto nitrocellulose membranes. A polyclonal antiserum raised against human CPR (5) was used as primary antibody, and a donkey anti-rabbit horseradish peroxidase IgG as secondary antibody (Scottish Antibody Production, Carluke, United Kingdom). Immunoreactivity was determined by chemiluminiscence (ECL Plus, Amersham-Pharmacia, Amersham, UK) and XAR5 autoradiographic film (Eastman Kodak). Glutathione levels were measured by the method of Sen et al.(6) and cytochrome P450 reductase activity was determined as previously described (5).

### Blood Chemistry

Blood was collected by cardiac puncture into heparinised tubes, and serum prepared by centrifugation. Serum was either analysed immediately or stored at -70 °C for a period not exceeding 4 weeks. Analysis for serum alanine aminotransferase and cholesterol was carried out using commercially available kits (Infinity Reagents, Sigma, Poole, UK) on a Cobas Fara II centrifugal analyser (Roche, UK).

### Histopathology

Tissue samples were either fixed in formalin-PBS for 24 h and transferred to 80% ethanol for storage, or snap-frozen embedded in OCT on cork discs and stored at -70 °C. Formalin-fixed samples were sectioned and stained with haematoxylin and eosin, or processed for immunostaining with a polyclonal antibody against rat CPR or various P450s. Snap-frozen tissue samples were cryosectioned and processed for staining with Oil Red O to determine lipid content.

### Statistical analysis

Statistical analysis was carried out using the Statview program (v4.5) for Macintosh, Abacus Concepts, Berkeley, CA,

### Isolation of mouse hepatocytes

Wild-type and hepatic CPR-null mice were anaesthetised by intraperitoneal injection of pentobarbitone (100mg/kg body weight) and the livers immediately perfused with Calcium-free Krebs-Ringer phosphate buffer, pH 7.4 (KRPB) *via* the hepatic portal vein at a flow rate of 12ml/min for 10 minutes followed by Calcium-free Krebs-Ringer hydrogen carbonate buffer, pH 7.4 (KRHB) at 12ml/min for 5 minutes and then 0.2 mg/ml collagenase in KRHB containing 0.3mM calcium chloride at 12ml/min for 10 minutes. Cell suspensions were then produced by mechanically disrupting removed liver tissue, filtration through bolting cloth and washing in KRHB.

### Hepatocyte cell culture

Mouse hepatocytes were grown at 37°C in complete Leibowitz L15 (CL-15) medium supplemented with 8.3% fetal calf serum (FCS), penicillin (41 IU / ml), streptomycin (41 µg /ml), tryptose phosphate broth (8.3%, v/v), hydrocortisone 21-hemisuccinate (10 µM), insulin (1 µM), hemin (5 µg/ml) and glutamine (240 µg / ml). Hepatocytes were seeded at a density of 8 x 10⁵ cells per well on 6-well plates and incubated at 37°C for one day before infection with human cytochrome P450 3A4-human cytochrome P450 reductase fusion gene by adenoviral vector.

### Ad-3A4/red plasmid construction and virus preparation

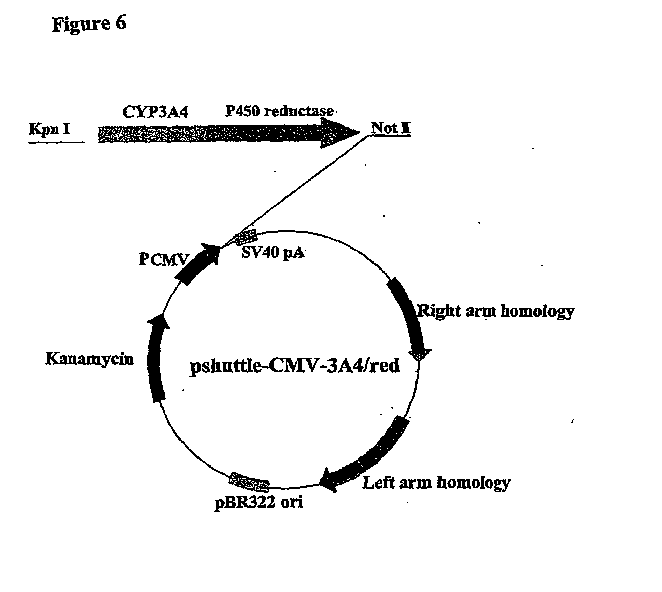

The human P450 3A4 (CYP3A4)-P450 reductase (CPR) fusion cDNA comprised contiguous modified CYP3A4 and CPR coding sequences. The CYP3A4 sequence was modified at the N-terminus by insertion of a *Kpn* I restriction site following the Kozak sequence and just before ATG start codon and by deletion of the TGA stop codon. The CPR sequence was modified by deletion of coding sequence for the first 57 amino acids (ER anchor) and flanked at the C-terminus by a *NotI* restriction site. The CYP3A4-CPR fusion cDNA was inserted into the shuttle vector pshuttle-CMV (Stratagene) please see Figure 6 and Ad-3A4/red virus prepared using the AdEasy XL Adenoviral vector system (Stratagene).

### Virus infection and determination of catalytic activity in cultured cells

Hepatocytes were infected with Ad-3A4/red adenovirus according to methods of the AdEasy XL Adenoviral vector system (Stratagene). After 4-days of infection with virus, the cell medium were replaced with 2 ml of fresh CL-15 plus bufuralol (10 µM or midazolam (10 µM). After 0.5, 1, 2 and 4 hours of incubation, 200 µl aliquots of medium were transferred to Eppendorf tubes with 200 µl ice-cold acetonitrile containing 5 µM dextrorphan as internal standard for mass spectrometric analysis.

### Mass Spectrometric analysis for bufuralol, midazolam and specific metabolites

Analysis for the loss of bufuralol and midazolam was carried out using reverse-phase HPLC with tandem mass spectrometric detection (LC-MS/MS). An aliquot of each standard and sample (20µL) was injected onto the liquid chromatography system and eluted under isocratic conditions of 50% H₂O with 0.1 %(v/v) Formic acid and 50% Acetonitrile at a flow rate of 0.5mL/min through a Phenomenex MercuryMS Luna 3µM C18(2), 20x2mm HPLC column.

Positive ions for parent compounds and specific fragment products were monitored in Multiple Reaction Monitoring (MRM) mode using a Micromass Quattro Micro Mass Spectrometer with Micromass MassLynx software version 3.5, monitoring for the positive bufuralol parent ion at 262.41 m/z with the specific product ion at 188.12 m/z; for the positive midazolam parent ion at 326.35 m/z with the specific product ion at 291.22 m/z and for internal standard dextrorphan parent ion at 258.47 m/z with the specific product ion at 157.21 m/z.

Analysis for the appearance of the specific metabolites 1'-hydroxybufuralol and 1'-hydroxymidazolam was carried out as above, but using gradient elution from the HPLC column and MRM of the positive 1'-hydroxybufuralol parent ion at 278.4 m/z with the specific product ion at 186.2 m/z and of the positive 1'-hydroxymidazolam parent ion at 342.3 m/z with the specific product ion at 203.2 m/z.

### EXAMPLE 1

### Generation of conditional CPR knockout mice

CPR is a multi-domain protein containing NADPH/ flavinadeninedinucleotide (FAD) and flavinmononucleotide (FMN) binding domains. When expressed in *E. coli,* these domains fold to form a functional polypeptide, which can, in the case of the FAD domain, independently catalyse the one electron reduction of a number of foreign compounds. For this reason, we made a construct, covering exons 3 to 16 of the CPR gene, where both of these domains would be deleted. (Fig. 1*a*). This construct was transfected into embryonic stem cells and a number of mouse lines generated. The correct integration of the floxed CPR targeting construct was confirmed by Southern analysis (Fig. 1*b*) of genomic DNA isolated from CPR^{lox/+} mice (lanes 1 and 5 (9.2kb/8kb bands), while the pattern obtained from a wild-type mouse (CPR^{+/+}) is shown in lane 2 (8kb band only). Genomic DNA of mice generated from ES cells in which Cre recombinase had been transiently expressed, resulted in deletion of the CPR gene between the first and third loxP sites. (Fig. 1*b*) lane 6 (CPR^{+/-}, 8kb/3.2kb bands). Mice homozygous for the floxed CPR locus (CPR^{lox/lox}) or with one floxed and one deleted CPR allele (CPR^{lox/-}) were generated by mating of appropriate mouse lines. Southern analysis of genomic DNA from these mice is shown in lanes 7 and 8 (CPR^{lox/lox}, 9.2kb band only) and from in lanes 3 and 4 CPR^{lox/-}, 9.2kb/3.2kb). These latter mice were apparently completely normal, displaying no phenotypic differences from wild-type littermates: their growth and development, blood chemistry, organ size and structure, and fertility were identical (data not shown).

### EXAMPLE 2

### Generation of hepatic CPR mull mice

In order to test the ability of Cre recombinase to inactivate the CPR allele, mice carrying CPR^{lox/-} were injected with a modified adenovirus in which Cre expression is regulated by the cytomegalovirus (CMV) promotor. One week after a single intravenous injection of AdCre1, hepatic CPR expression was reduced by >95% as measured by immunoblotting of hepatic microsomes and immunostaining of liver sections (not shown).

Specific hepatic deletion of CPR was achieved by crossing CPR^{lox/lox} mice into a line where Cre expression was regulated by the rat albumin promotor. Mice identified as CPR^{lox/+} + Cre^{ALB} were either backcrossed with CPR^{lox/lox} mice to generate a CPR^{lox/lox} + Cre^{ALB} line, or crossed with CPR heterozygous nulls to generate CPR^{lox/-} + Cre^{ALB} mice. The presence of the Cre^{ALB} transgene was determined by PCR (data not shown). Offspring born from either of these crosses were found in Mendelian proportions as predicted from parental genotype, indicating there was no embryonic lethality, from opportunistic expression of the Cre transgene during development.

CPR^{lox/-} + Cre^{ALB} and CPR^{lox/lox} + Cre^{ALB} mice displayed no overt phenotypic differences from their wild-type littermates in the postnatal period: mice of these genotypes grew and developed normally. As the albumin promotor becomes active neonatally, we investigated hepatic CPR levels in mice of 6-8 weeks of age. As mice were heterozygous for the Cre^{ALB} genotype, three genotypes were anticipated, ie where no, one or both CPR alleles were deleted. This indeed proved to be the case. Deletion of floxed alleles appeared to occur with high efficiency; in mice where one CPR allele had been deleted, only half the expression and activity of CPR was found in both males and females (Fig. 2*a*, liver, tracks 2 & 3 vs. 4 & 5, and tracks 7 & 8 vs. 9 & 10). In mice of genotype CPR^{lox/-} + Cre^{ALB}, an immunoreactive CPR protein band was essentially absent in both males and females (Fig. 2*a*, lanes 1 and 6, respectively), indicating an almost complete lack of CPR protein in the microsomal fractions of the livers of these animals. Upon prolonged exposure (>10-fold normal) of the immunoblot shown in Fig. 2*a*, a very faint band corresponding to the correct molecular weight for CPR could be seen (not shown). Hepatic CPR activity in CPR^{lox/-} + Cre^{ALB} mice was reduced by more than 90% in both males (92.5%) and females (94.5%) (Fig. 2*b*). The activity of cytochrome b5 reductase, an enzyme which could conceivably transfer electrons from NADH to the P450 system, was unchanged (not shown). In liver sections from CPR hepatic null mice stained with a polyclonal antiserum to CPR, only a very few cells contained immunoreactive protein (Fig. 2*c*), in contrast to the wild-type mice where CPR immunostaining was extensive across the entire section, tending to be more concentrated in the perivenous regions in a zonal fashion as previously reported. The deletion of CPR using a genetic strategy means that hepatocytes with no CPR immunostaining cannot have any CPR activity. The very low residual CPR activity observed in hepatic microsomal fractions may be due to continued expression of CPR in cells other than hepatocytes and/or in cells where the albumin promotor (and thus Cre expression) is inactive. The specificity of the CPR deletion was demonstrated by showing that no change was observed in CPR expression in the kidneys of CPR^{lox/+} + Cre^{ALB}, CPR^{lox/+} and CPR^{+/+} + Cre^{ALB} mice in both sexes (Fig. 2a, Kidney; lanes 3,4 & 5 (male) and 8, 9 & 10 (female)). A reduction in CPR protein level of approximately 50% was seen in CPR^{lox/-} + Cre^{ALB} and in CPR^{lox/-} mice was observed (Fig. 2*a*, lanes 1 & 2 (male) and 6 & 7 (female)) in offspring missing only one CPR allele. Again, protein expression reflected the CPR activity (Fig. 2b) in kidney microsomal fractions, with both male and female CPR^{lox/-} + Cre^{ALB} mice exhibiting approximately half the CPR activity of CPR^{+/+} + Cre^{ALB} mice. Further confirmation of the tissue-specific nature of the CPR deletion was demonstrated by immunostaining kidney and lung sections from CPR^{lox/-} + Cre^{ALB} and CPR^{+/+} + Cre^{ALB} mice with CPR antiserum (Fig. 2*c*), where the staining was the same in both lines.

### EXAMPLE 3

### Characterisation of hepatic CPR null mice

Mice nulled for hepatic CPR exhibited no overt phenotypic differences to controls. The mice grew at the same rate as their wild-type counterparts and there was no change in survival rates or behaviour. In spite of the known activity of the hepatic P450 monooyxgenase system in the metabolism of androgens and oestrogens, CPR hepatic null mice exhibited normal fertility when mated to wild-type (CPR^{+/+} or CPR^{lox/lox}) mice, indicating that hepatic hormone metabolism does not affect fertility.

More detailed analysis showed that both male and female hepatic CPR nulls displayed hepatomegaly, the liver being approximately 50% larger, in relation to body weight (7.5%), compared to wild-type mice (4%). Furthermore, the liver was pale in colour, and the tissue was mottled and friable (Fig. *3a**,c*)*.* Microscopic examination revealed the presence of microvisicular and macrovisicular fatty changes, the former predominating (Fig. 3*b*, plates *i* and *iv* vs. *iii* and *vii*)*.* Otherwise, hepatocytes in both the centrizonal and periportal regions appeared normal, with no apparent increase in hepatocyte proliferation or apoptosis.

The livers of mice lacking hepatic CPR were hyperlipidaemic, in terms of both cholesterol and triglycerides, as evidenced by staining with SR19 (not shown) or Oil Red O (Fig. 3*b*, plates *ii* and *vi* vs. *iv* and *viii*)*.* The increase in hepatic cholesterol was accompanied by the almost complete absence of bile acids in the gall bladder. This phenotypic observation could be explained by the inactivation of P450s such as CYP7A1 (cholesterol 7α-hydroxylase), reportedly the rate-limiting in bile acid biospthesis²¹⁻²³ In the absence of cholesterol breakdown, we would have anticipated that an increase in circulating cholesterol would occur; however, exactly the opposite was observed, with serum cholesterol levels being reduced dramatically (>65%) (Fig. 3*c*). The control of cholesterol homeostasis is complex, however, and the above data demonstrate that the microsomal P450 system plays a key role in determining circulating cholesterol levels. A deletion of CYP7A1 has been described²¹, which exhibited increased postnatal mortality but essentially unchanged serum cholesterol levels²². The fact that increased mortality was not observed in this study was probably due to the conditional nature of the CPR deletion, occurring after the neonatal period, and reaching a peak after 3-4 weeks (not shown). It is interesting to note that the CVP7A1^{-/-} mice were reported to have similar serum cholesterol levels to wild-type mice, and in order to assess the extent to which the accumulation of hepatic cholesterol in hepatic CPR null mice was due to *de novo* synthesis, these mice were placed on a fat-free diet for up to 3 months. Under these conditions, the observed changes in hepatic and serum cholesterol were only partially reversed (not shown).

Under normal circumstances, cholesterol accumulation in the liver would trigger the feedback suppression of cholesterol uptake and biosynthesis by inhibiting the action of the sterol regulatory element binding protein (SREBP) transcription factors on a number of key lipogenic genes such as HMGCoA reductase, HMGCoA synthase and squalene synthase, and also the LDL receptor. It is interesting to note that transgenic mice expressing a truncated 'dominant positive' form of SRBBP1 (7) displayed a similar hepatomegaly and elevation of hepatic lipid content as found here. However, these mice had essentially normal serum lipid chemistry.

The above data suggest that the P450 system plays additional important roles in regulating circulating cholesterol levels and that inhibition of these enzymes can reduce, rather than increase, cholesterol in the serum. Hepatic CPR nulls also demonstrated a marked increase in hepatic triglyceride levels, with a corresponding decrease in serum triglycerides (Fig. 3*c*). Although CYP4A proteins are involved in fatty acid metabolism²⁶⁻²⁸, it is unlikely that the profound increase observed could be explained by the absence of this activity, indicating a potentially novel function of CPR and/or the P450 system in regulating lipid levels. No change in hepatic peroxisomal activity, often associated with CYP4A and fatty acid accumulation, was observed in hepatic CPR nulls (not shown). Counter intuitively, circulating triglyceride levels were reduced by 25% (CPR^{lox/-} + Cre^{ALB}) and 70% (CPR^{lox/lox} + Cre^{ALB}) in serum of reductase null mice relative to controls (Fig. 3*c*)*.*

### EXAMPLE 4

### P450 activity in hepatic CPR null mice

In order to determine the effect of CPR deletion on hepatic P450 monoxygenase activities, we measured the microsomal hydroxylation of testosterone (Fig. 4*a*). When expressed as pmol metabolite per nmol P450, a 90% reduction in the formation of 6β-hydroxytestosterone, an activity associated with CYP3A proteins, was measured in both males and females, indicating that a lack of CPR in this tissue resulted in severely compromised P450 function. The 7α-hydroxylation of testosterone, carried out by P450s of the CYP2A subfamily, was decreased by >99% in both males and females, while 16β-hydroxylation was undetectable in hepatic CPR nulls of both sexes (Fig. 4*a*). In addition to the marked reduction in testosterone metabolism, the O-dealkylation of 7-methoxyresorufin was also almost completely ablated in CPR^{lox/-} + Cre^{ALB} mice, being reduced by 99.5% in males and 98.5% in females, when expressed nmol metabolite per nmol P450 (not shown). It is thus clear that hepatic deletion of CPR results in the inactivation of multiple P450s in the liver.

Intriguingly, mice lacking hepatic CPR exhibited a profound increase in cytochrome P450 content; in males the increase was 450%, while in females it was greater than 500% (Fig. 4*b*). This large increase in P450 expression was further demonstrated by immunoblotting of hepatic microsomes with antisera to P450s from different gene families (Fig. 4c*)*. For certain of the enzymes, eg members of CYP2B and CYP3A gene families, the induction was extremely large. Indeed, these P450s were induced to at least as high a level as observed when potent exogenous P450 inducers are administered to mice. Hepatic immunostaining clearly showed that while expression of CYP3A P450s was confined to the perivenous or centrilobular area in wild-type mice, in null mice CYP3A was localised throughout the section at high levels (Fig. 4*d*). These novel and fundamental observations demonstrate that endogenous factors, in addition to those identified as mediating the sexual differentiation of the murine P450 system can profoundly affect hepatic cytochrome P450 content. Why this occurs is however unclear. In the absence of P450 activity, endogenous inducing agents which are substrates for these enzymes, (e.g. glucocorticoids) may accumulate; alternatively, P450 turnover may be reduced. The rationale for this is based on the observation that CPR is the electron donor to haem oxygenase and increased CPR expression in transfected cells reduces P450 levels. Finally, there may be a novel compensatory regulatory response to the absence of P450 activity.

### EXAMPLE 5

### In vivo phenotypes

The analgesic drug paracetamol (acetaminophen) is activated by cytochrome P450s to a highly reactive hepatotoxic intermediate, N-acetylbenzoquinonimine (NAPQI), which can be detoxified by conjugation with glutathione; overwhelming this inactivation pathway results in hepatotoxicity, with fatal consequences. In order to unequivocally demonstrate the role of the P450 system in these effects, acetaminophen was administered as a single intraperitoneal dose to male control and hepatic CPR null mice (Fig. 5*a, b*)*.* In controls, a 90% decrease in hepatic glutathione occurred within one hour of administration (Fig. 5*a*), whereas the level remained unchanged in CPR nulls. Furthermore, 24h after treatment, wild-type mice showed a marked rise in serum ALT, indicative of extensive, probably fatal, liver damage, whereas in mice lacking hepatic CPR ALT remained unchanged.

Cytochrome P450s involved in drug metabolism are distributed throughout the tissues of the body; it has been estimated that a significant proportion of drug metabolism (up to 30%) may be extra-hepatic. In order to test the level of extra-hepatic metabolism as well as the *in vivo* effects of hepatic CPR nulls on drug disposition, we measured barbiturate-induced sleeping times. This method has been used since the 1960s as an indicator of drug metabolising activity. In controls, administration of pentobarbitone (20 mg/kg) failed to induce sleep; however, mice lacking hepatic CPR, of genotype CPR^{3lox/-} + Cre or CPR^{3lox/3lox} + Cre^{ALB}, slept for a period in excess of 2 hours. Pentobarbitone treatment of CPR heterozygous null mice (CPR^{+/-}), ie lacking one CPR allele and therefore having approximately 50% less CPR activity, resulted in an average sleeping period of approximately 50 minutes, illustrating a clear gene-dosage effect, and also that CPR activity is rate-limiting *in vivo* for the metabolism of this substrate. The above profound changes in drug response show that extra-hepatic metabolism plays only a minor role in the disposition of this compound. The data also demonstrate that the potential alternate electron transport pathway for P450s, from NADH via cytochrome b5/b5 reductase, plays only a minimal role, if any, in drug disposition.

One of the most intriguing aspects of the animals of the present invention is that through the deletion of a single gene the functions of an entire multi-gene family has been ablated in a tissue-specific manner. In the case of the liver, the entire P450 system has essentially been inactivated, resulting in a profound reduction in bile acid production and drug metabolism. In spite of the hepatic phenotype, hepatic CPR null mice live (over 18 months) and reproduce normally. This extraordinary finding means that the hepatic P450 system in adults is not essential for life and furthermore accentuates its role in protecting mammals from toxic environmental agents. Currently, the hepatic metabolism of endogenous molecules such as steroid hormones does not appear to be of major significance in endogenous hormone homeostasis. However, the P450 system does play a major role in regulating lipid homeostasis and hepatic lipid levels. The animals of the present invention, tissues and/or cells derived therefrom, methods of their production and uses all as hereinbefore described here will allow fundamental questions, many of which have been unanswered for several decades, finally to be addressed.

### EXAMPLE 6

### Expression of Human Cytochrome P450 3A4 Activity in Isolated Hepatocytes from Hepatic CPR-null Mice

In this study, isolated hepatocytes from hepatic CPR-null mice were infected with adenovirus carrying a human cytochrome P450 3A4-cytochrome P450 reductase fusion gene under control of the CMV promoter. This resulted in expression of human P450 3A4 activity in the infected hepatocytes showing that cells from the CPR-null animals can be used as the basis of 'humanised' metabolic systems.

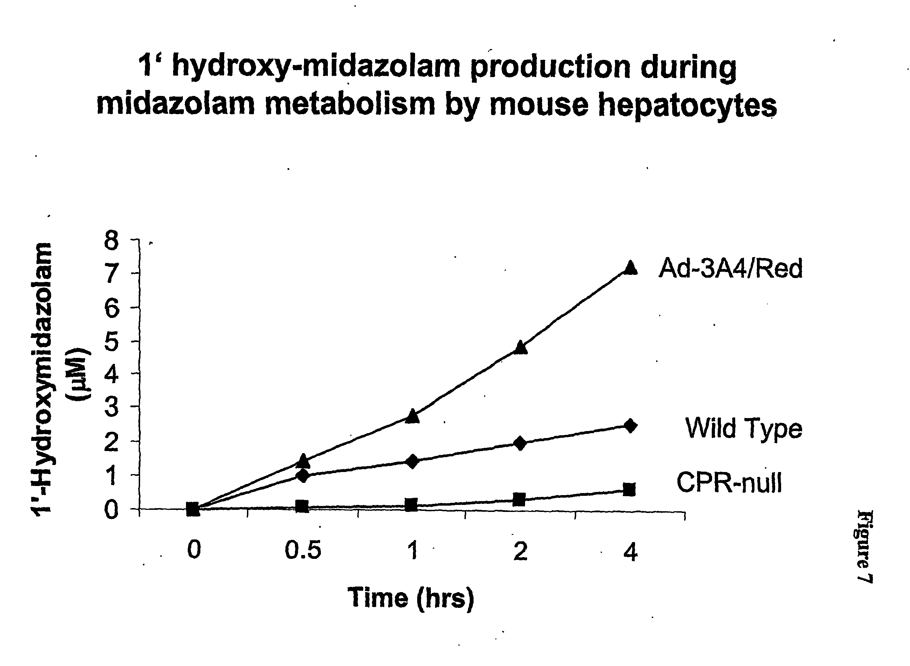

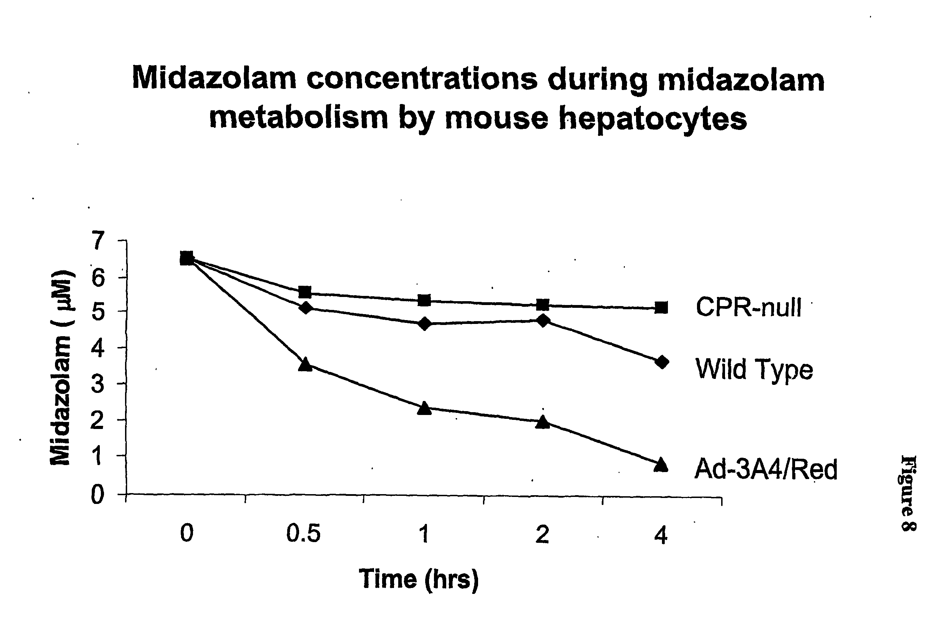

In order to determine the effect of human recombinant CYP3A4/reductase on hepatic CPR-null hepatocytes P450 monoxygenase activities, the catalytic activity of the CYP3A4 was measured using midazolam and bufuralol, monitoring disappearance of the added drugs and accumulation of the metabolites l'hydroxy-midazolam and l'hydroxy-bufuralol in hepatocytes from wild-type mice and hepatocytes from hepatic CPR-null mice with or without infection with the Ad-3A4/red fusion vector. After 4 hours incubation, l'hydroxy-midazolam concentrations in hepatocyte cultures from hepatic CPR-null mice incubated with midazolam were only approximately 25% of those produced in cultures from wild-type mice. However, cultures from CPR-null mice that had been infected with Ad-3A4/red produced approximately 3 times as much l'hydroxy-midazolam as those from wild-type animals as can be seen in the graph represented by Figure 7. Corresponding effects were seen on the disappearance of midazolam as is apparent from Figure 8.

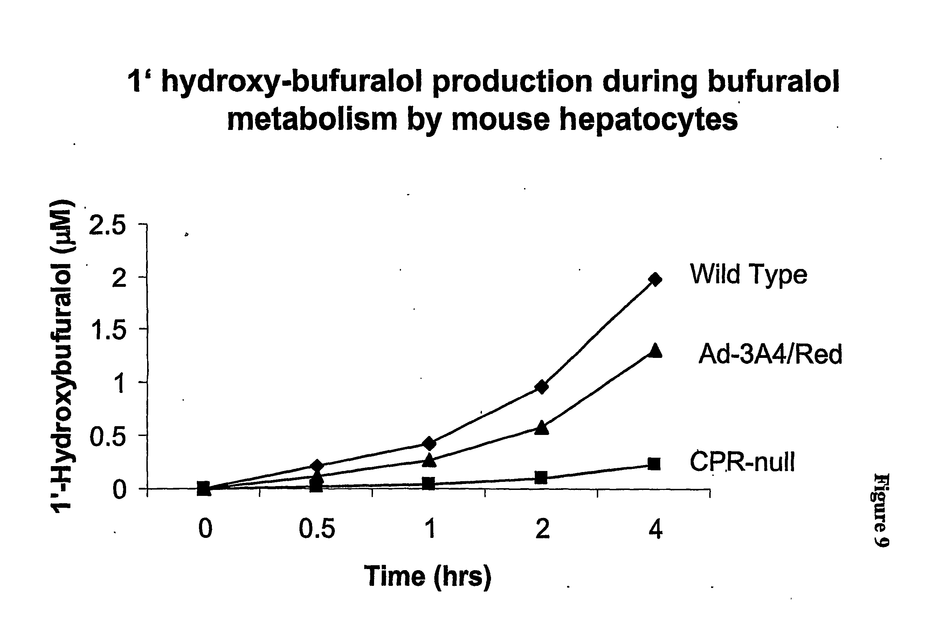

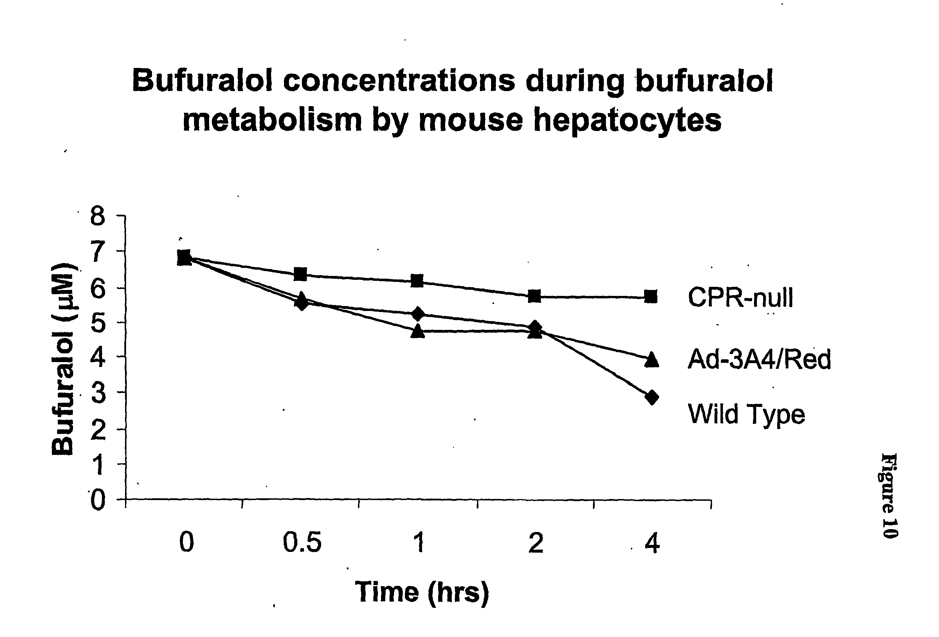

Similarly, 4 hours incubation with bufuralol resulted in l'hydroxy-bufuralol concentrations in hepatocyte cultures from hepatic CPR-null mice that were only approximately 10% of those produced in cultures from wild-type mice, while concentrations of l'hydroxy-bufuralol in cultures from CPR-null mice infected with Ad-3A4/red were approximately 65% of those from wild-type animals (Figure 9). Corresponding effects were seen on the disappearance of bufuralol as can be seen in the graph of Figure 10.

These results show that expression of the CYP3A4-CPR fusion protein restores cytochrome P450-mediated metabolism to hepatocytes from CPR-null animals and, moreover, that at least some of this is due to expression of the human cytochrome P450 3A4 activity as shown by differing degrees of restoration of function with respect to midazolam and bufuralol metabolism.

## Claims

1. Use of a non-human transgenic animal comprising cells with a conditional deletion of hepatic cytochrome P450 reductase gene in selecting chemical compounds suitable for development as drugs and other products wherein the non-human transgenic animal is selected from the group comprising monkey, dog, cat, rabbit, hamster, rat and mouse.

2. Use of a non-human transgenic animal comprising cells with a conditional deletion of hepatic cytochrome P450 reductase gene in designing chemical compounds suitable for development as drugs and other products wherein the non-human transgenic animal is selected from the group comprising monkey, dog, cat, rabbit, hamster, rat and mouse..

3. Use of a non-human transgenic animal comprising cells with a conditional deletion of hepatic cytochrome P450 reductase gene in determining tissue disposition of a drug or other chemical compound wherein the non-human transgenic animal is selected from the group comprising monkey, dog, cat, rabbit, hamster, rat and mouse..

4. Use of a non-human transgenic animal comprising cells with a conditional deletion of hepatic cytochrome P450 reductase gene in toxicity studies wherein the non-human transgenic animal is selected from the group comprising monkey, dog, cat, rabbit, hamster, rat and mouse.

5. Use of a non-human transgenic animal comprising cells with a conditional deletion of cytochrome hepatic P450 reductase gene in studying the contribution of cytochrome P450s to the aetiology of disease states wherein the non-human transgenic animal is selected from the group comprising monkey, dog, cat, rabbit, hamster, rat and mouse.

6. Use of a non-human transgenic animal comprising cells with a conditional deletion of hepatic cytochrome P450 reductase gene in studying the contribution of cytochrome P450s in pregnancy and/or neonatal development wherein the non-human transgenic animal is selected from the group comprising monkey, dog, cat, rabbit, hamster, rat and mouse.

7. Use of a non-human transgenic animal comprising cells with a conditional deletion of hepatic cytochrome P450 reductase gene in studying the contribution of cytochrome P450s in modulation of hormone function and/or hormone levels and other endogenous pathways in which P450s play a role wherein the non-human transgenic animal is selected from the group comprising monkey, dog, cat, rabbit, hamster, rat and mouse.

8. Use of a non-human transgenic animal comprising cells with a conditional deletion of hepatic cytochrome P450 reductase gene in studying the contribution of cytochrome P450s in substrate metabolism wherein the non-human transgenic animal is selected from the group comprising monkey, dog, cat, rabbit, hamster, rat and mouse.

9. Use according to either claim 7 or 8 wherein the hormone/substrate is selected from the group comprising: steroids, estrogens, androgens, prostacyclins, cholesterol, glucocorticoids, arachadonic acid, thromboxane, prostaglandin, Vitamin D3, neurosteroids, catechols, bile acid, fatty acids or retinoic acid.

10. Use according to claim 5 wherein the disease states are selected from the group comprising: choleostasis, atherogenesis, hormonal imbalances, neurological disorders, degenerative diseases, skin, conditions, cardiovascular disease, cancer or glaucoma.

11. Use of a non-human transgenic animal comprising cells with a conditional deletion of hepatic cytochrome P450 reductase gene in identifying products which reduce circulating cholesterol levels wherein the non-human transgenic animal is selected from the group comprising monkey, dog, cat, rabbit, hamster, rat and mouse.

12. Use according to any preceding claim wherein the animal is embryonic, neonatal, juvenile, adolescent and/or adult.

13. Use of non-human animal cells having a conditional deletion of hepatic cytochrome P450 reductase gene as an *in vitro* screen in product discovery, development and/or disposition/toxicity studies and/or rationalised drug design.

14. Use of non-human animal cells having a conditional deletion of hepatic cytochrome P450 reductase gene as an *in vitro* screen in studying disease states, pregnancy, neonatal development and/or modulation of hormone function and/or hormone levels and or other endogenous pathways and substrate metabolism in which cytochrome P450s play a role.

15. Use of non-human animal cells having a floxed cytochrome P450 reductase locus as an *in vitro* screen in product development/discovery and/or disposition/toxicity studies and/or rationalised drug design, in studying disease states, pregnancy or neonatal development and/or modulation of hormone function and/or hormone levels and or other endogenous pathways and substrate metabolism in which cytochrome P450s play a role

16. Use according to any one of claims 13 to 15 wherein the cells are in the form of a tissue.

17. Use according to any preceding claim wherein the animal is a mouse and/or animal cells are murine.

18. Use according to any one of claims 1 to 8 or 11 or 13 to 15 wherein the animal is obtainable by or has the characteristics of an animal produced by, a method comprising the steps of transfecting non-human embryonic stem cells with a cytochrome P450 reductase (CPR) targeting construct, selecting animals homozygous for a floxed CPR locus (CPR ^{lox/lox}) and crossing said animals with a line where Cre expression is regulated.

19. Use according to claim 18 wherein regulation of Cre expression is performed by a tissue or cell specific promoter or where expression is constitutive or is inducible/regulatable by administration of an exogenous agent.

20. Use according to claim 19 wherein Cre expression is inducible by administration of tamoxifen or tetracycline.

21. Use according to either claim 19 or 20 wherein the promotor is selected from the group comprising a rat albumin promoter, CMV promoter, an intestinal fatty acid binding protein promoter or a bovine keratin VI promoter or any other promotor that is activated in a tissue specific manner by a conditional response.

22. Use according to claim 19 wherein the tissue or cell specific promoter is selected from the group comprising a cytochrome P450 promoter, a promoter of another drug metabolism enzyme or a transcriptional factor promoter.

23. Use according to claim 22 wherein the cytochrome P450 promoter is CYP1A1, CYP3A, CYP4A, CYP2B1 or CYP2B6.

24. Use according to claim 22 wherein the transcriptional factor promoter is PXR or Nrf2.

25. Use according to any of claims 18 to 24 wherein the CPR targeting construct includes exons 3 to 16 of the CPR gene.

26. Use according to claim 25 wherein the construct comprises a 12 kb *SalI* fragment.

27. Use according to either claim 25 or 26 wherein the construct is flanked by same-orientation *loxP* sites and included in a cassette comprising a promoter and optionally further includes a selectable marker.

28. Use according to claim 27 wherein the marker is neomycin.

29. Use according to either claim 27 or 28 wherein the promoter is a herpes simplex thymidine kinase (*hsv-tk*) promotor.

30. Use according to any one of claims 26 to 29 wherein the cassette is inserted into intron 4.

31. Use of a construct as defined in any one of claims 25 to 30 in the production of a non human transgenic animal comprising cells with a conditional deletion of hepatic cytochrome P450 reductase gene or animal cells with a conditional deletion of hepatic cytochrome P450 reductase gene.

32. Use according to any one of claims 1 to 8 or 11 or 13 to 15 wherein the animal is obtainable by, or has the characteristics of, an animal produced by, a method comprising the steps of transfecting non-human embryonic stern cells with a site specific recombinase system target for a suitably modified cytochrome P450 reductase gene, selected from the group comprising Flp/ftr, C31/attB, attP or a gene trap vector recombinase system and selecting animals homozygous for said deletion.

33. Use according to any one of claims 1 to 17 wherein a method of deleting or ablating the cytochrome P450 reductase gene or reducing/preventing its expression products comprises siRNA technology to specifically target a gene's product thereby resulting in null or hypomorphic phenotypes.

34. Use of a cytochrome P450 reductase null or reductase null heterozygote non-human transgenic animal selected from the group comprising monkey, dog, cat, rabbit, hamster, rat and mouse as an *in vivo* screen to determine any one or more of the following parameters:
(i) role of cytochrome P450s or cytochrome P450 reductase in product disposition or of disposition of any other organic, small weight molecule;
(ii) pathways of product disposition;
(iii) routes of Phase II drug disposition and/or toxicity of a parent product;
(iv) role of drug transporters in drug uptake;
(v) role of cytochrome P450s or P450 reductases in pathways of chemical toxicity;
(vi) role of P450 system in normal metabolic processes, homeostasis, disease models or as a drug/product target; or
(vii) role of P450 system in pathogenesis or etiology of diseases.

35. Use of a cytochrome P450 reductase null or reductase null heterozygote non-human transgenic animal selected from the group comprising monkey, dog, cat, rabbit, hamster, rat and mouse as an *in vivo* screen to determine any one or more of the following parameters:
(i) pharmacological potency of agents subject to first pass metabolism;
(ii) occurrence and rate of extra-hepatic metabolism;
(iii) rate of metabolic clearance as a determinant in product distribution and pharmacokinetics;
(iv) assessment of lead product selection based on *in vivo* metabolic parameters;
(v) cross into drug discovery models so as to establish effect of metabolism on pharmacological activity and therapeutic outcome;
(vi) assessment of relevance and occurrence of pharmaco-logically active metabolites;
(vii) distinguish between toxicity due to metabolic activation or other mechanisms;
(viii) establish role of P450 or P450 reductase as a rate limiting step in drug/product disposition;
(ix) role of bile flow on product elimination or;
(x) study drug/drug interactions due either to P450 or drug transporter effects.

36. Use of a cytochrome P450 reductase null or reductase null heterozygote non-human transgenic animal selected from the group comprising monkey, dog, cat, rabbit, hamster, rat and mouse in any one or more of the following uses:
(i) as a cross into a disease model so as to establish the role of metabolism in pharmacological/toxicological effects;
(ii) as a cross into a multidrug response protein (Mdr1 and Mdr2) null mouse to evaluate a dual role of both P450s and Mdr in compound bioavailability studies;
(iii) as a cross into a reporter animal so as to study toxicological response(s) thereto, product induction and/or pharmacological response thereto, to establish the role of the P450 system or P450 reductase in that response;
(iv) in generating cell lines with no P450 reductase activity; or
(v) in generating novel *in vivo* mutation tests so as to evaluate the role of P450/P450 reductase in chemical mutagenesis.

37. Use according to any of claims 34 to 36 wherein the non-human transgenic animal is a hepatic reductase null or hepatic reductase null heterozygote.

38. Use of non-human transgenic animal tissues and/or cells with a conditional deletion of hepatic cytochrome P450 reductase gene as an *in vitro* assay for the study of any one or more of the following events/parameters:
(i) role of transporters in compound uptake and efflux;
(ii) identification of compound metabolites produced by the cytochrome P450 system;
(iii) evaluation of candidate compounds as P450 substrates;
(iv) assessment of drug/drug interactions due to P450 effects;
(v) cross into multi-drug resistant (Mdr) null animals so as to evaluate a dual role of P450s and Mdr in drug bioavailability;
(vi) use of an S9 fraction in an Ames test so as to determine potential P450-mediated product mutagenicity;
(vii) establish involvement of the cytochrome P450 system in hepatotoxicity/carcinogenicity;
(viii) establish whether hepatic product metabolism or metabolism of any other P450 containing cell is involved in toxicity to other organs;
(ix) establish whether hepatic or any other cell production of a compound metabolite (i.e. pro-drug activation) is important for pharmacological action/toxicity of a given candidate product;
(x) establish whether a parent product or its P450 generated metabolite(s) mediate induction of drug metabolising enzymes, liver growth or other phenotypic effects;
(xi) investigate role of the P450 system in peroxisome proliferation;
(xii) investigate role of cytochrome P450 reductase in product disposition, toxicity or metabolic activation or;
(xiii) investigate presence of secondary sites of toxicity in the absence of toxicity in any cell type due to P450-mediated product metabolism.

39. A transgenic non-human animal selected from the group comprising monkey, dog, cat, rabbit, hamster, rat and mouse, comprising cells with a conditional deletion of hepatic cytochrome P450 reductase gene, tissues and/or cells from said non-human transgenic animal that have a conditional deletion of hepatic cytochrome P450 reductase gene, the said non-human transgenic animal, tissue and or cells being further modified so as to contain or express DNA encoding a human cytochrome P450 or another protein involved in drug metabolism.

## Patentansprüche

1. Verwendung eines nicht menschlichen transgenen Tiers, welches Zellen mit einer konditionalen Deletion des hepatischen Cytochrom P450-Reduktase-Gens umfasst, bei der Auswahl chemischer Verbindungen, welche geeignet für die Entwicklung als Arzneien und andere Produkte sind, wobei das nicht menschliche transgene Tier aus der Affe, Hund, Katze, Kaninchen, Hamster, Ratte und Maus umfassenden Gruppe ausgewählt wird.

2. Verwendung eines nicht menschlichen transgenen Tiers, welches Zellen mit einer konditionalen Deletion des hepatischen Cytochrom P450-Reduktase-Gens umfasst, bei der Konzeption chemischer Verbindungen, welche geeignet für die Entwicklung als Arzneien und andere Produkte sind, wobei das nicht menschliche transgene Tier aus der Affe, Hund, Katze, Kaninchen, Hamster, Ratte und Maus umfassenden Gruppe ausgewählt wird.

3. Verwendung eines nicht menschlichen transgenen Tiers, welches Zellen mit einer konditionalen Deletion des hepatischen Cytochrom P450-Reduktase-Gens umfasst, bei der Bestimmung der Gewebedisposition einer Arznei oder anderen chemischen Verbindung, wobei das nicht menschliche transgene Tier aus der Affe, Hund, Katze, Kaninchen, Hamster, Ratte und Maus umfassenden Gruppe ausgewählt wird.

4. Verwendung eines nicht menschlichen transgenen Tiers, welches Zellen mit einer konditionalen Deletion des hepatischen Cytochrom P450-Reduktase-Gens umfasst, bei Toxizitätsstudien, wobei das nicht menschliche transgene Tier aus der Affe, Hund, Katze, Kaninchen, Hamster, Ratte und Maus umfassenden Gruppe ausgewählt wird.

5. Verwendung eines nicht menschlichen transgenen Tiers, welches Zellen mit einer konditionalen Deletion des hepatischen Cytochrom P450-Reduktase-Gens umfasst, bei der Untersuchung des Anteils von Cytochromen P450-Genen an der Ätiologie von Krankheitszuständen, wobei das nicht menschliche transgene Tier aus der Affe, Hund, Katze, Kaninchen, Hamster, Ratte und Maus umfassenden Gruppe ausgewählt wird.

6. Verwendung eines nicht menschlichen transgenen Tiers, welches Zellen mit einer konditionalen Deletion des hepatischen Cytochrom P450-Reduktase-Gens umfasst, bei der Untersuchung des Anteils von Cytochromen P450-Genen bei Schwangerschaft und/oder neonataler Entwicklung, wobei das nicht menschliche transgene Tier aus der Affe, Hund, Katze, Kaninchen, Hamster, Ratte und Maus umfassenden Gruppe ausgewählt wird.

7. Verwendung eines nicht menschlichen transgenen Tiers, welches Zellen mit einer konditionalen Deletion des hepatischen Cytochrom P450-Reduktase-Gens umfasst, bei der Untersuchung des Anteils von Cytochromen P450-Genen bei der Modulation der Hormonfunktion und/oder Hormonspiegeln und anderer endogener Wege, bei denen P450-Gene eine Rolle spielen, wobei das nicht menschliche transgene Tier aus der Affe, Hund, Katze, Kaninchen, Hamster, Ratte und Maus umfassenden Gruppe ausgewählt wird.

8. Verwendung eines nicht menschlichen transgenen Tiers, welches Zellen mit einer konditionalen Deletion des hepatischen Cytochrom P450-Reduktase-Gens umfasst, bei der Untersuchung des Anteils von Cytochromen P450-Genen beim Substratmetabolismus, wobei das nicht menschliche transgene Tier aus der Affe, Hund, Katze, Kaninchen, Hamster, Ratte und Maus umfassenden Gruppe ausgewählt wird.

9. Verwendung nach Anspruch 7 oder 8, wobei das Hormon/Substrat aus der Folgendes umfassenden Gruppe ausgewählt wird: Steroide, Estrogene, Androgene, Prostacycline, Cholesterin, Glucocorticoide, Arachidonsäure, Thromboxan, Prostaglandin, Vitamin D3, Neurosteroide, Brenzcatechine, Gallensäure, Fettsäuren oder Retinolsäure.

10. Verwendung nach Anspruch 5, wobei die Krankheitszustände aus der Folgendes umfassenden Gruppe ausgewählt werden: Cholestase, Atherogenese, Hormon-Ungleichgewichte, neurologische Funktionsstörungen, degenerative Krankheiten, Hautleiden, Herz-Kreislauf-Erkrankung, Krebs oder Glaukom.

11. Verwendung eines nicht menschlichen transgenen Tiers, welches Zellen mit einer konditionalen Deletion des hepatischen Cytochrom P450-Reduktase-Gens umfasst, bei der Ermittlung von Produkten, welche zirkulierende Cholesterinspiegel reduzieren, wobei das nicht menschliche transgene Tier aus der Affe, Hund, Katze, Kaninchen, Hamster, Ratte und Maus umfassenden Gruppe ausgewählt wird.

12. Verwendung nach einem beliebigen vorangehenden Anspruch, wobei das Tier embryonal, neonatal, juvenil, adoleszent und/oder erwachsen ist.

13. Verwendung nicht menschlicher Tierzellen mit einer konditionalen Deletion des hepatischen Cytochrom P450-Reduktase-Gens als *in vitro-*Screen bei der Auffindung und Entwicklung von Produkten und/oder Dispositions-/Toxizitätsstudien und/oder rationalisiertem Wirkstoffdesign.

14. Verwendung nicht menschlicher Tierzellen mit einer konditionalen Deletion des hepatischen Cytochrom P450-Reduktase-Gens als *in vitro-*Screen bei der Untersuchung von Krankheitszuständen, Schwangerschaft, neonataler Entwicklung und/oder der Modulation von Hormonfunktionen und/oder Hormonspiegeln und/oder anderen endogenen Wegen und Substratmetabolismus, bei denen Cytochrom P450-Gene eine Rolle spielen.

15. Verwendung nicht menschlicher Tierzellen mit einem gefloxten Cytochrom P450 Reduktase-Locus als *in vitro-*Screen bei der Produktentwicklung/-auffindung und/oder Dispositions-/Toxizitätsstudien und/oder rationalisiertem Wirkstoffdesign, bei der Untersuchung von Krankheitszuständen, Schwangerschaft oder neonataler Entwicklung und/oder der Modulation von Hormonfunktionen und/oder Hormonspiegeln und/oder anderen endogenen Wegen und Substratmetabolismus, bei denen Cytochrom P450-Gene eine Rolle spielen.

16. Verwendung nach einem beliebigen der Ansprüche 13 bis 15, wobei die Zellen in Form von Gewebe vorliegen.

17. Verwendung nach einem beliebigen vorangehenden Anspruch, wobei das Tier eine Maus ist und/oder Tierzellen von Mäusen stammen.

18. Verwendung nach einem beliebigen der Ansprüche 1 bis 8 oder 11 oder 13 oder 15, wobei das Tier durch ein Verfahren erhältlich ist oder die Eigenschaften eines durch ein solches Verfahren erhaltenen Tieres aufweist, wobei dieses Verfahren die Schritte der Transfektion nicht menschlicher embryonaler Stammzellen mit einem Cytochrom P450 Reduktase (CPR)-Targeting-Konstrukt, die Auswahl homozygoter Tiere für einen gefloxten CPR-Locus (CPR^{lox/flox}) und Kreuzen der genannten Tiere mit einer Linie umfasst, bei der die Cre-Expression reguliert ist.

19. Verwendung nach Anspruch 18, wobei die Regulation der Cre-Expression durch einen gewebe- oder zellspezifischen Promotor erfolgt oder wo die Expression konstitutiv ist oder durch die Verabreichung eines exogenen Mittels induzierbar/regulierbar ist.

20. Verwendung nach Anspruch 19, wobei die Cre-Expression durch Verabreichung von Tamoxifen oder Tetracyclin induzierbar ist.

21. Verwendung nach Anspruch 19 oder 20, wobei der Promotor aus der Folgendes umfassenden Gruppe ausgewählt wird: einen Rattenalbumin-Promotor, CMV-Promotor, Promotor intestinale Fettsäure-bindenden Proteins oder einen Promotor des Rinder-Keratins VI oder einen beliebigen anderen Promotor, welcher auf eine gewebespezifische Weise durch eine bedingte Reaktion aktiviert wird.

22. Verwendung nach Anspruch 19, wobei der gewebe- oder zellenspezifische Promotor aus der Folgendes umfassenden Gruppe ausgewählt wird: einen Cytochrom P450-Promotor, einen Promotor eines anderen Arzneimittelstoffwechsel-Enzyms oder einen Transkriptionsfaktor-Promotor.

23. Verwendung nach Anspruch 22, wobei der Cytochrom P450-Promotor CYP1 A1, CYP3A, CYP4A, CYP2B1 oder CYP2B6 ist.

24. Verwendung nach Anspruch 22, wobei der Transkriptionsfaktor-Promotor PXR oder Nrf2 ist.

25. Verwendung nach einem beliebigen der Ansprüche 18 bis 24, wobei das CPR-Targeting-Konstrukt Exons 3 bis 16 des CPR-Gens umfasst.

26. Verwendung nach Anspruch 25, wobei das Konstrukt ein 12 kb langes Sall-Fragment umfasst.

27. Verwendung nach Anspruch 25 oder 26, wobei das Konstrukt durch loxP-Sites gleicher Orientierung flankiert und in einer einen Promotor umfassenden Kassette enthalten ist und ferner einen selektierbaren Marker umfassen kann.

28. Verwendung nach Anspruch 27, wobei der Marker Neomycin ist.

29. Verwendung nach Anspruch 27 oder 28, wobei der Promotor ein Herpes Simplex-Thymidinkinase (*hsv*-*tk*)-Promotor ist.

30. Verwendung nach einem beliebigen der Ansprüche 26 bis 29, wobei die Kassette in Intron 4 eingesetzt wird.

31. Verwendung eines Konstrukts, wie es in einem beliebigen der Ansprüche 25 bis 30 definiert ist, beim Erhalt eines nicht menschlichen transgenen Tieres mit Zellen mit einer konditionalen Deletion des hepatischen Cytochrom P450-Reduktase-Gens oder von Tierzellen mit einer konditionalen Deletion des hepatischen Cytochrom P450-Reduktase-Gens.

32. Verwendung nach einem beliebigen der Ansprüche 1 bis 8 oder 11 oder 13 oder 15, wobei das Tier nach einem Verfahren erhaltbar ist oder die Eigenschaften eines durch ein solches erhaltenen Tieres aufweist, wobei dieses Verfahren die Schritte der Transfektion nicht menschlicher embryonaler Stammzellen mit einem sitespezifischen RekombinaseSystem-Target für ein geeignet modifiziertes Cytochrom P450 Reduktase-Gen umfasst, welches aus der Flp/ftr, C31/attB, attP oder ein Gen-Trap-Vektor-Rekombinasesystem umfassenden Gruppe ausgewählt wird, sowie die Auswahl homozygoter Tiere für die genannte Deletion umfasst.

33. Verwendung nach einem beliebigen der Ansprüche 1 bis 17, wobei ein Verfahren zur Deletion oder Ablation des Cytochrom P450-Reduktase-Gens oder zur Verringerung/Verhinderung seiner Expressionsprodukte siRNA-Technologie umfasst, um das Produkt eines Gens spezifisch zu targetieren, wodurch sich Null- oder hypomorphe Phänotypen ergeben.

34. Verwendung eines Cytochrom P450-Reduktase-Null- oder Reduktase-Null- heterozygoten nicht menschlichen transgenen Tieres, welches aus der Affe, Hund, Katze, Kaninchen, Hamster, Ratte und Maus umfassenden Gruppe ausgewählt wird, als *in vivo*-Screen bei der Bestimmung eines beliebigen oder mehr der folgenden Parameter:
(i) Rolle von Cytochrom P450s oder Cytochrom P450-Reduktase bei der Disposition des Produkts oder der Disposition eines beliebigen anderen organischen Moleküls von geringem Gewicht;
(ii) Wege der Disposition des Produkts;
(iii) Routen der Phase II-Arzneimitteldisposition und/oder Toxizität eines Vorgängerprodukts;
(iv) Rolle von Arzneimitteltransportern bei der Arzneimittelaufnahme;
(v) Rolle von Cytochrom P450s oder P450-Reduktasen bei Wegen chemischer Toxizität;
(vi) Rolle eines P450-Systems bei normalen metabolischen Prozessen, Homöostase, Krankheitsmodellen oder als Arzneimittel-/Produkt-Target; oder
(vii) Rolle eines P450-Systems bei der Pathogenese oder Ätiologie von Krankheiten.

35. Verwendung eines Cytochrom P450-Reduktase-Null- oder Reduktase-Null- heterozygoten nicht menschlichen transgenen Tieres, welches aus der Affe, Hund, Katze, Kaninchen, Hamster, Ratte und Maus umfassenden Gruppe ausgewählt wird, als in vivo-Screen bei der Bestimmung eines beliebigen oder mehr der folgenden Parameter:
(i) pharmakologische Potenz von Mitteln, die dem First-Pass-Metabolismus unterliegen;
(ii) Auftreten und Rate von extrahepatischem Metabolismus;
(iii) Stoffwechsel-Clearance-Rate als Bestimmungsgröße bei der Produktverteilung und Pharmakokinetik;
(iv) Festsetzung der Auswahl von Hauptentwicklungsprodukten auf der Grundlage von *in vivo*-Stoffwechselparametern;
(v) Querverbindung zu Arzneimittelentwicklungsmodellen, um die Wirkung des Stoffwechsels auf pharmakologische Aktivität und therapeutisches Ergebnis zu ermitteln;
(vi) Festsetzung der Relevanz und des Auftretens pharmakologisch aktiver Metaboliten;
(vii) Unterscheidung zwischen Toxizität aufgrund von Stoffwechselaktivierung oder anderen Mechanismen;
(viii) Ermitteln der Rolle von P450 oder P450-Reduktase als geschwindigkeitsbestimmender Schritt bei der Arzneimittel-/Produktdisposition;
(ix) Rolle des Gallenflusses bei der Produkteliminierung oder;
(x) Untersuchtung von Wechselwirkungen zwischen Arzneimitteln aufgrund von P450- oder Arzneimitteltransporter-Wirkungen.

36. Verwendung eines Cytochrom P450-Reduktase-Null- oder Reduktase-Null- heterozygoten nicht menschlichen transgenen Tieres, welches aus der Affe, Hund, Katze, Kaninchen, Hamster, Ratte und Maus umfassenden Gruppe ausgewählt wird, bei einer beliebigen oder mehr der folgenden Anwendungen:
(i) als Querverbindung zu einem Krankheitsmodell, um die Rolle des Stoffwechsels bei pharmakologischen/ toxikologischen Wirkungen zu ermitteln;
(ii) als Querverbindung zu einer Multidrug-Response-Protein (Mdr-1 und Mdr-2)-Null-Maus, um eine duale Rolle sowohl von P450s als auch von Mdr bei Verbindungs-Bioverfügbarkeitsstudien zu bewerten;
(iii) als Querverbindung zu einem Reportertier, um die toxikologische(n) Reaktion(en) darauf, Produktinduktion und/oder pharmakologische Reaktion darauf zu untersuchen, um die Rolle des P450-Systems oder P450-Reduktase bei dieser Reaktion zu ermitteln;
(iv) bei der Erzeugung von Zelllinien mit keiner P450-Reduktase-Aktivität; oder
(v) bei der Schaffung neuartiger *in vivo*-Mutationstests, um die Rolle von P450/P450-Reduktase bei der chemischen Mutagenese zu beurteilen.

37. Verwendung nach beliebigen der Ansprüche 34 bis 36, wobei das nicht menschliche transgene Tier ein hepatische Reduktase-Null oder hepatische Reduktase-Null-Heterozygot ist.

38. Verwendung nicht menschlicher transgener Tiergewebe und/oder-zellen mit einer konditionalen Deletion des hepatischen Cytochrom P450-Reduktase-Gens als *in vitro*-Assay zur Untersuchung eines beliebigen oder mehr der folgenden Ereignisse/Parameter:
(i) Rolle von Transportern bei Verbindungsaufnahme und -ausscheidung;
(ii) Ermittlung von zusammengesetzten Metaboliten, welche von dem Cytochrom P450-System erzeugt werden;
(iii) Bewertung von Kandidatenverbindungen als P450-Substrate;
(iv) Beurteilung von Wechselwirkungen zwischen Arzneimitteln aufgrund von P450-Wirkungen;
(v) Querverbindung zu Multidrug-Resistant (Mdr)-Null-Tieren, um eine duale Rolle von P450s und Mdr bei der Bioverfügbarkeit von Arzneimitteln zu bewerten;
(vi) Verwendung einer S9-Fraktion bei einem Ames-Test, um eine potentielle durch P450 herbeigeführte Produkt-Mutagenität zu bestimmen;
(vii) Bestimmung der Beteiligung des Cytochrom P450-Systems bei Hepatotoxizität/Carcinogenität;
(viii) Bestimmung, ob der Leberprodukt-Stoffwechsel oder der Stoffwechsel einer beliebigen anderen P450 enthaltenden Zelle an der Toxizität für andere Organe beteiligt ist;
(ix) Bestimmung, ob die Erzeugung eines zusammengesetzten Metaboliten (d.h. Prodrug-Aktivierung) durch die Leber oder einen beliebigen anderen Zelltyp bedeutend für die pharmakologische Wirkung/Toxizität eines bestimmten Produktkandidaten ist;
(x) Bestimmung, ob ein Vorgängerprodukt oder sein(e) durch P450 erzeugter/erzeugten Metabolit(en) die Induktion Arzneimittel metabolisierender Enzyme, Leberwachstum oder andere phänotypische Wirkungen herbeiführen;
(xi) Untersuchung der Rolle des P450-Systems bei der peroxisomalen Proliferation;
(xii) Untersuchung der Rolle von Cytochrom P450-Reduktase bei der Produktdisposition, Toxizität oder Stoffwechselaktivierung oder;
(xiii) Untersuchung des Vorhandenseins sekundärer Toxizitäts-Sites bei Abwesenheit von Toxizität in einem beliebigen Zelltyp aufgrund durch P450 herbeigeführten Produktstoffwechsels.

39. Nicht menschliches transgenes Tier, welches aus der Affe, Hund, Katze, Kaninchen, Hamster, Ratte und Maus umfassenden Gruppe ausgewählt wird und welches Zellen mit einer konditionalen Deletion des hepatischen Cytochrom P450-Reduktase-Gens umfasst, Gewebe und/oder Zellen des genannten nicht menschlichen transgenen Tieres, welche eine konditionale Deletion des hepatischen Cytochrom P450-Reduktase-Gens aufweisen, wobei das genannte nicht menschliche transgene Tier, Gewebe und/oder Zellen weiter modifiziert werden, so dass sie DNA enthalten oder exprimieren, welche ein menschliches Cytochrom P450 oder anderes beim Arzneimittelstoffwechsel beteiligtes Protein kodiert.

## Revendications

1. - Utilisation d'un animal transgénique non humain comprenant des cellules avec une délétion conditionnelle du gène de la cytochrome P450 réductase hépatique dans la sélection de composés chimiques appropriés pour un développement en tant que médicaments et autres produits, l'animal transgénique non humain étant choisi dans le groupe comprenant le singe, le chien, le chat, le lapin, le hamster, le rat et la souris.

2. - Utilisation d'un animal transgénique non humain comprenant des cellules avec une délétion conditionnelle du gène de la cytochrome P450 réductase hépatique dans la conception de composés chimiques appropriés pour un développement en tant que médicaments et autres produits, l'animal transgénique non humain étant choisi dans le groupe comprenant le singe, le chien, le chat, le lapin, le hamster, le rat et la souris.

3. - Utilisation d'un animal transgénique non humain comprenant des cellules avec une délétion conditionnelle du gène de la cytochrome P450 réductase hépatique dans la détermination de la mise à disposition tissulaire d'un médicament ou autre composé chimique, l'animal transgénique non humain étant choisi dans le groupe comprenant le singe, le chien, le chat, le lapin, le hamster, le rat et la souris.

4. - Utilisation d'un animal transgénique non humain comprenant des cellules avec une délétion conditionnelle du gène de la cytochrome P450 réductase hépatique dans des études de toxicité, l'animal transgénique non humain étant choisi dans le groupe comprenant le singe, le chien, le chat, le lapin, le hamster, le rat et la souris.

5. - Utilisation d'un animal transgénique non humain comprenant des cellules avec une délétion conditionnelle du gène de la cytochrome P450 réductase hépatique dans l'étude de la contribution des cytochromes P450 à l'étiologie d'états de maladie, l'animal transgénique non humain étant choisi dans le groupe comprenant le singe, le chien, le chat, le lapin, le hamster, le rat et la souris.

6. - Utilisation d'un animal transgénique non humain comprenant des cellules avec une délétion conditionnelle du gène de la cytochrome P450 réductase hépatique dans l'étude de la contribution des cytochromes P450 dans la grossesse et/ou le développement néonatal, l'animal transgénique non humain étant choisi dans le groupe comprenant le singe, le chien, le chat, le lapin, le hamster, le rat et la souris.

7. - Utilisation d'un animal transgénique non humain comprenant des cellules avec une délétion conditionnelle du gène de la cytochrome P450 réductase hépatique dans l'étude de la contribution des cytochromes P450 dans la modulation de la fonction hormonale et/ou des taux hormonaux et d'autres voies endogènes dans lesquelles les P450 jouent un rôle, l'animal transgénique non humain étant choisi dans le groupe comprenant le singe, le chien, le chat, le lapin, le hamster, le rat et la souris.

8. - Utilisation d'un animal transgénique non humain comprenant des cellules avec une délétion conditionnelle du gène de la cytochrome P450 réductase hépatique dans l'étude de la contribution des cytochromes P450 dans le métabolisme d'un substrat, l'animal transgénique non humain étant choisi dans le groupe comprenant le singe, le chien, le chat, le lapin, le hamster, le rat et la souris.

9. - Utilisation selon l'une des revendications 7 ou 8, dans laquelle l'hormone/le substrat est choisi dans le groupe comprenant : les stéroïdes, les oestrogènes, les androgènes, les prostacyclines, le cholestérol, les glucocorticoïdes, l'acide arachidonique, le thromboxane, la prostaglandine, la vitamine D3, les neurostéroïdes, les catéchols, l'acide biliaire, les acides gras ou l'acide rétinoïque.

10. - Utilisation selon la revendication 5, dans laquelle les états de maladie sont choisis dans le groupe comprenant : une choléostase, une athérogenèse, des déséquilibres hormonaux, des troubles neurologiques, des maladies dégénératives, des états cutanés, des maladies cardiovasculaires, un cancer ou un glaucome.

11. - Utilisation d'un animal transgénique non humain comprenant des cellules avec une délétion conditionnelle du gène de la cytochrome P450 réductase hépatique dans l'identification de produits qui réduisent les taux de cholestérol circulants, l'animal transgénique non humain étant choisi dans le groupe comprenant le singe, le chien, le chat, le lapin, le hamster, le rat et la souris.

12. - Utilisation selon l'une quelconque des revendications précédentes, dans laquelle l'animal est embryonnaire, nouveau-né, juvénile, adolescent et/ou adulte.

13. - Utilisation de cellules animales non humaines ayant une délétion conditionnelle du gène de la cytochrome P450 réductase hépatique en tant que crible *in vitro* dans la découverte, le développement et/ou des études de mise à disposition/toxicité de produits et/ou une conception de médicament rationalisée.

14. - Utilisation de cellules animales non humaines ayant une délétion conditionnelle du gène de la cytochrome P450 réductase en tant que crible *in vitro* dans l'étude d'états de maladie, d'une grossesse, du développement néonatal et/ou de la modulation de la fonction hormonale et/ou des taux d'hormone et/ou d'autres voies endogènes et métabolismes de substrat dans lesquels les cytochromes P450 jouent un rôle.

15. - Utilisation de cellules animales non humaines ayant un locus de cytochrome P450 réductase floxé en tant que crible *in vitro* dans le développement/la découverte et/ou des études de mise à disposition/toxicité de produits et/ou une conception de médicament rationalisée, dans l'étude d'états de maladie, d'une grossesse ou du développement néonatal et/ou d'une modulation de la fonction hormonale et/ou des taux hormonaux et/ou d'autres voies endogènes et métabolismes de substrat dans lesquels les cytochromes P450 jouent un rôle.

16. - Utilisation selon l'une quelconque des revendications 13 à 15, dans laquelle les cellules sont sous la forme d'un tissu.

17. - Utilisation selon l'une quelconque des revendications précédentes, dans laquelle l'animal est une souris et/ou les cellules animales sont murines.

18. - Utilisation selon l'une quelconque des revendications 1 à 8 ou 11 ou 13 à 15, dans laquelle l'animal peut être obtenu par, ou a les caractéristiques d'un animal produit par, un procédé comprenant les étapes de transfection de cellules souches embryonnaires non humaines par un produit de construction ciblant la cytochrome P450 réductase (CPR), la sélection d'animaux homozygotes pour un locus CPR floxé (CPR^{lox/lox}) et le croisement desdits animaux avec une lignée dans laquelle l'expression de Cre est régulée.

19. - Utilisation selon la revendication 18, dans laquelle la régulation de l'expression de Cre est réalisée par un promoteur spécifique d'un tissu ou d'une cellule, ou dans laquelle l'expression est constitutive ou est inductible/régulable par l'administration d'un agent exogène.

20. - Utilisation selon la revendication 19, dans laquelle l'expression de Cre est inductible par l'administration de tamoxifène ou de tétracycline.

21. - Utilisation selon l'une des revendications 19 ou 20, dans laquelle le promoteur est choisi dans le groupe comprenant un promoteur d'albumine de rat, un promoteur CMV, un promoteur de protéine intestinale de liaison aux acides gras, ou un promoteur de kératine VI bovine ou tout autre promoteur qui est activé d'une manière spécifique d'un tissu par une réponse conditionnelle.

22. - Utilisation selon la revendication 19, dans laquelle le promoteur spécifique d'un tissu ou d'une cellule est choisi dans le groupe comprenant un promoteur de cytochrome P450, un promoteur d'une autre enzyme de métabolisme de médicament ou un promoteur de facteur de transcription.

23. - Utilisation selon la revendication 22, dans laquelle le promoteur de cytochrome P450 est CYP1A1, CYP3A, CYP4A, CYP2B1 ou CYP2B6.

24. - Utilisation selon la revendication 22, dans laquelle le promoteur de facteur de transcription est PXR ou Nrf2.

25. - Utilisation selon l'une quelconque des revendications 18 à 24, dans laquelle le produit de construction ciblant CPR comprend les exons 3 à 16 du gène CPR.

26. - Utilisation selon la revendication 25, dans laquelle le produit de construction comprend un fragment de 12 kb de *Sall.*

27. - Utilisation selon l'une des revendications 25 ou 26, dans laquelle le produit de construction est flanqué par des sites *loxP* d'orientation identique et compris dans une cassette comprenant un promoteur et comprend facultativement en outre un marqueur sélectionnable.

28. - Utilisation selon la revendication 27, dans laquelle le marqueur est la néomycine.

29. - Utilisation selon l'une des revendications 27 ou 28, dans laquelle le promoteur est un promoteur de thymidine kinase d'herpès simplex (*hsv*-tk).

30. - Utilisation selon l'une quelconque des revendications 26 à 29, dans laquelle la cassette est insérée dans l'intron 4.

31. - Utilisation d'un produit de construction tel que défini à l'une quelconque des revendications 25 à 30 dans la production d'un animal transgénique non humain comprenant des cellules avec une délétion conditionnelle du gène de la cytochrome P450 réductase hépatique ou de cellules animales avec une délétion conditionnelle du gène de la cytochrome P450 réductase hépatique.

32. - Utilisation selon l'une quelconque des revendications 1 à 8 ou 11 ou 13 à 15, dans laquelle l'animal peut être obtenu par, ou a les caractéristiques d'un animal produit par un procédé comprenant les étapes de transfection de cellules souches embryonnaires non humaines par un système de recombinase spécifique d'un site, cible d'un gène de cytochrome P450 réductase modifié de façon appropriée, choisi dans le groupe comprenant Flp/ftr, C31/attB, attP ou un système de recombinase vecteur de piège génique, et de sélection d'animaux homozygotes pour ladite délétion.

33. - Utilisation selon l'une quelconque des revendications 1 à 17, dans laquelle un procédé de délétion ou d'ablation du gène de la cytochrome P450 réductase ou de réduction/prévention de ses produits d'expression comprend une technologie d'ARNsi pour cibler de façon spécifique un produit de gène, permettant ainsi d'obtenir des phénotypes nuls ou hypomorphes.

34. - Utilisation d'un animal transgénique non humain nul pour la cytochrome P450 réductase ou hétérozygote nul pour la réductase, choisi dans le groupe comprenant le singe, le chien, le chat, le lapin, le hamster, le rat et la souris en tant que crible *in vivo* pour déterminer l'un quelconque ou plusieurs des paramètres suivants :
(i) le rôle des cytochromes P450 ou de la cytochrome P450 réductase dans la mise à disposition d'un produit ou la mise à disposition de tout autre molécule organique à faible masse ;
(ii) les voies de mise à disposition d'un produit ;
(iii) les voies de mise à disposition d'un médicament de Phase II et/ou la toxicité d'un produit parent ;
(iv) le rôle de transporteurs de médicaments dans l'absorption d'un médicament ;
(v) le rôle des cytochromes P450 ou P450 réductases dans les voies de toxicité chimique ;
(vi) le rôle du système P450 dans les processus métaboliques normaux, l'homéostase, les modèles de maladie ou en tant que cible de médicament/produit ; ou
(vii) le rôle du système P450 dans la pathogenèse ou l'étiologie de maladies.

35. - Utilisation d'un animal transgénique non humain nul pour la cytochrome P450 réductase ou hétérozygote nul pour la réductase choisi dans le groupe comprenant le singe, le chien, le chat, le lapin, le hamster, le rat et la souris en tant que crible *in vivo* pour déterminer l'un quelconque ou plusieurs des paramètres suivants :
(i) la puissance pharmacologique d'agents soumis à un métabolisme de premier passage ;
(ii) la survenue et la vitesse du métabolisme extrahépatique ;
(iii) la vitesse de clairance métabolique en tant que déterminant dans la distribution et la pharmacocinétique d'un produit;
(iv) l'évaluation d'une sélection d'un produit principal sur la base de paramètres métaboliques *in vivo* ;
(v) le croisement dans des modèles de découverte de médicament de façon à établir l'effet du métabolisme sur l'activité pharmacologique et le résultat thérapeutique ;
(vi) l'évaluation de la pertinence et de la survenue de métabolites actifs pharmacologiquement ;
(vii) le fait de distinguer entre une toxicité due à une activation métabolique ou à d'autres mécanismes ;
(viii) l'établissement du rôle de P450 ou de P450 réductase en tant qu'étape limitant la vitesse dans la mise à disposition du médicament/du produit ;
(ix) le rôle du flux biliaire sur l'élimination du produit ou ;
(x) l'étude d'interactions médicament/médicament dues soit à P450 soit aux effets de transporteurs de médicaments.

36. - Utilisation d'un animal transgénique non humain nul pour la cytochrome P450 réductase ou hétérozygote nul pour la réductase, choisi dans le groupe comprenant le singe, le chien, le chat, le lapin, le hamster, le rat et la souris dans l'une quelconque ou plusieurs des utilisations suivantes :
(i) en tant que croisement dans un modèle de maladie de façon à établir le rôle du métabolisme dans des effets pharmacologiques/toxicologiques ;
(ii) en tant que croisement dans une souris nulle pour une protéine de réponse multimédicaments (Mdr1 et Mdr2) pour évaluer un rôle double d'à la fois les P450 et Mdr dans des études de biodisponibilité de composé ;
(iii) en tant que croisement dans un animal rapporteur pour étudier la ou les réponses toxicologiques sur celui-ci, l'induction de produit et/ou la réponse pharmacologique à celui-ci, pour établir le rôle du système P450 ou de la P450 réductase dans cette réponse ;
(iv) dans la génération de lignées cellulaires sans activité P450 réductase ; ou
(v) dans la génération de nouveaux tests de mutation *in vivo,* de façon à évaluer le rôle de P450/P450 réductase dans la mutagenèse chimique.

37. - Utilisation selon l'une quelconque des revendications 34 à 36, dans laquelle l'animal transgénique non humain est un animal nul pour la réductase hépatique ou hétérozygote nul pour la réductase hépatique.

38. - Utilisation de tissus et/ou cellules animaux transgéniques non humains avec une délétion conditionnelle du gène de la cytochrome P450 réductase hépatique en tant qu'essai *in vitro* pour l'étude de l'un quelconque ou plusieurs des évènements/paramètres suivants :
(i) le rôle de transporteurs dans l'absorption et l'efflux d'un composé ;
(ii) l'identification de métabolites d'un composé produits par le système cytochrome P450 ;
(iii) l'évaluation de composés candidats en tant que substrats de P450 ;
(iv) l'évaluation d'interactions de médicament/médicament dues à des effets P450 ;
(v) le croisement dans des animaux nuls pour une résistance multimédicaments (Mdr) de façon à évaluer un rôle double des P450 et Mdr dans la biodisponibilité de médicament ;
(vi) l'utilisation d'une fraction S9 dans un test d'Ames de façon à déterminer la mutagénicité potentielle d'un produit à médiation par P450;
(vii) l'établissement de l'implication du système cytochrome P450 dans l'hépatotoxicité/la carcinogénicité ;
(viii) l'établissement du fait de savoir si le métabolisme hépatique d'un produit ou le métabolisme de toute autre cellule contenant P450 est impliqué dans la toxicité envers d'autres organes ;
(ix) l'établissement du fait de savoir si la production hépatique ou par tout autre cellule d'un métabolite de composé (c'est-à-dire activation d'un promédicament) est importante pour l'action pharmacologique/la toxicité d'un produit candidat donné ;
(x) l'établissement du fait de savoir si un produit parent ou son ou ses métabolites générés par P450 assure la médiation de l'induction d'enzymes de métabolisation de médicament, d'une croissance hépatique ou d'autres effets phénotypiques ;
(xi) l'investigation du rôle du système P450 dans la prolifération des peroxysomes ;
(xii) l'investigation du rôle de la cytochrome P450 réductase dans la mise à disposition d'un produit, la toxicité ou l'activation métabolique ; ou
(xiii) l'investigation de la présence de sites secondaires de toxicité en l'absence de toxicité dans tout type cellulaire due au métabolisme d'un produit à médiation par P450.

39. - Animal transgénique non humain choisi dans le groupe comprenant le singe, le chien, le chat, le lapin, le hamster, le rat et la souris, comprenant des cellules avec une délétion conditionnelle du gène de cytochrome P450 réductase hépatique, tissus et/ou cellules provenant dudit animal transgénique non humain qui ont une délétion conditionnelle du gène de la cytochrome P450 réductase hépatique, lesdits animal transgénique non humain, tissus et/ou cellules étant de plus modifiés de façon à contenir ou à exprimer l'ADN codant pour un cytochrome P450 humain ou pour une autre protéine mise en jeu dans le métabolisme de médicament.
